# EUROPÄISCHE PATENTSCHRIFT

(11) **EP 0 391 212 B1**
(45) Veröffentlichungstag und Bekanntmachung des Hinweises auf die Patenterteilung: **25.05.1994**
(21) Anmeldenummer: 90105805.7
(22) Anmeldetag: 27.03.1990
(51) Int. Cl.: C07C 63/66, C08F 12/08, C07C 69/76

(54) **Verfahren zur Herstellung von bifunktionellen Z-Stilbenverbindungen, neue bifunktionelle Z-Stilbenverbindungen sowie die Verwendung der Z-Stilbenverbindungen zur Herstellung von Polymeren**
Process for preparing bifunctional Z-stilbene compounds, new bifunctional Z-stilbene compounds and their use for preparing polymers
Procédé de préparation de composés de stilbène bifonctionnels ayant la configuration Z, composés de stilbène ayant cette configuration ainsi que leur utilisation pour la préparation de polymères

(30) Priorität: 07.04.1989 DE 3911221
(43) Veröffentlichungstag der Anmeldung: 10.10.1990
(73) Patentinhaber: BAYER AG, 51368 Leverkusen (DE)
(72) Erfinder: Arlt, Dieter, Prof. Dr., D-5000 Köln 80 (DE); Bader, Axel, Dr., D-5060 Bergisch Gladbach 2 (DE); Eckhardt, Volker, Dr., D-4150 Krefeld (DE)

(56) Entgegenhaltungen:
- EP-A- 0 040 581
- HOUBEN-WEYL: "Methoden der Organische Chemie", 4. Auflage, Band XIII/9b: A.SEGNITZ: "Metallorganische Verbindungen", 1984, Seiten 987-991,Georg Thieme Verlag, Stuttgart, DE
- HOUBEN-WEYL: "Methoden der Organischen Chemie", 4. Auflage, Band V/2a: V.JÄGER: "Alkine, Di- und Polyine, Allene, Kumulene", 1977, Seiten405-406,649, Georg Thieme Verlag, Stuttgart, DE
- HOUBEN-WEYL: "Methoden der Organischen Chemie", 4. Auflage, Band V/1b: H.BALLI: "Alkene, Cycloalkene, Arylalkene", 1972, Seiten 588-591,Georg Thieme Verlag, Stuttgart, DE
- TETRAHEDRON LETTERS, Nr. 36, September 1978, Seiten 3323-3324; J.G. BATT et al.: "Synthesis and cycloaddition reactions of 2-triethylsilyl-1,3-butadiene"
- ORGANIC REACTIONS, Band 27, 1982, Seiten 345-389; R.F. HECK: "Palladium-catalyzed vinylation of organic halides"

## Beschreibung

Die Erfindung betrifft ein Verfahren zur Herstellung von bifunktionellen Z-Stilbenverbindungen, neue bifunktionelle Z-Stilbenverbindungen sowie die Verwendung der Z-Stilbenverbindungen zur Herstellung von Polymeren.

EP- A 40 581 offenbart Vinylstilbenverbindungen ohne Angabe der Konfiguration der Doppelbindung, ein Verfahren zu deren Herstellung sowie deren Verwendung als optische Aufheller.

Z(cis)-Stilbene lassen sich z.B. herstellen, indem man Arylaldehyde und Arylessigsäuren einer Perkin-Kondensation unterwirft und die gebildeten cis-α,β-Diarylacrylsäuren in Chinolin in Gegenwart von Kupferchromit bei Temperaturen von etwa 200°C decarboxyliert (vgl. F. Merger in Houben-Weyl, Bd. V/1b, S. 308). Dieses Verfahren liefert jedoch nicht immer reine Z-Stilbene, sondern die Produkte enthalten Anteile der entsprechenden E(trans)-Stilbene, deren Abtrennung durch Chromatographie oder Kristallisation aufwendig ist und nicht immer vollständig gelingt. Ein weiterer Nachteil des Verfahrens sind die zur Decarboxylierung erforderlichen hohen Temperaturen (ca. 200°C), die zu Nebenreaktionen Anlaß geben können. Die Ausbeuten sind mäßig. Die Variationsbreite der Reaktionsfolge ist durch die zum Teil schlechte Zugänglichkeit substituierter Arylaldehyde und Arylessigsäuren gering.

Die Synthese alkylsubstituierter Z-Stilbene wird beschrieben von E.B. Merkushev und T.S. Skorokhodova in Zhurnal Organicheskoi Khimii, Vol. 18, No. 2, pp. 355-359. Als Einsatzprodukte werden hierbei Alkyliodbenzole verwendet, deren Umsetzung mit Acetylen unter Pd-Katalyse Di(alkylaryl)acetylene liefert, die anschließend stereospezifisch zu Z-Stilbenen hydriert werden.

Nachteilig ist bei dem genannten Verfahren die Verwendung der teuren und schlecht zugänglichen Aryliodide. Die Reaktion wird ausschließlich für Iodbenzol oder alkylsubstituierte Iodbenzole beschrieben und eignet sich in der Hauptsache nur zur Darstellung der entsprechenden symmetrischen, dialkylsubstituierten Z-Stilbene.

Es wurde nun ein Verfahren zur Herstellung von bifunktionellen, mindestens eine Z-konfigurierte Stilbengruppierung enthaltenden Stilbenverbindungen der Formel 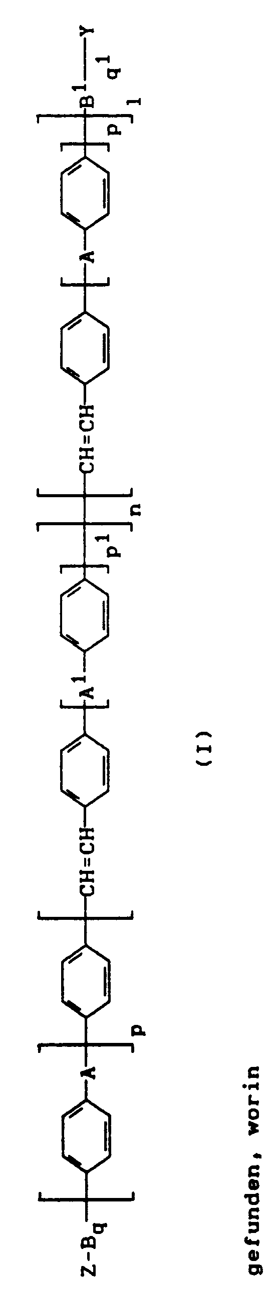
- Z und Y: gleich oder verschieden sind und für COOR, COR, COCl, OCOCl, NCO, NRCOCl, NHR, OR oder Cl stehen mit R = Wasserstoff oder C₁-C₈-Alkyl,
- A und A¹: gleich oder verschieden sind und für eine chemische Bindung oder CH=CH stehen,
- B: 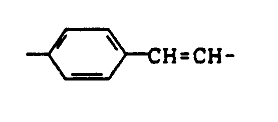 und
- B¹: 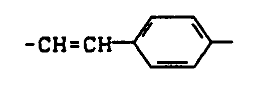 bedeuten,
- l: 0 oder 1,
- p und p¹: 0 oder 1,
- q und q¹: 0 oder 1 und
- n: 0, 1 oder 2 bedeuten,
das dadurch gekennzeichnet ist, daß man Verbindungen der Formel 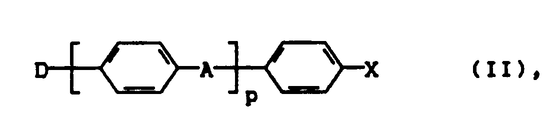
worin
- A und p: die obengenannte Bedeutung besitzen,
- D: COOR¹, COR¹, NHR², NO₂, OR³, Cl oder Br bedeutet mit R¹ = Wasserstoff oder verzweigtes oder geradkettiges C₁-C₈-Alkyl, R² = OAc, COOC₂H₅ oder eine andere geeignete Schutzgruppe und R³ = OAc, 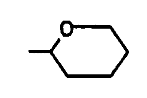 oder eine andere geeignete Schutzgruppe,
und
- X: für Brom oder Iod steht,
mit einem Acetylen der Formel

HC≡C-R⁴ (III),

worin
- R⁴: für 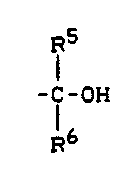 oder -Si(R⁵)₃ steht,
mit R⁵ und R⁶ = C₁-C₄-Alkyl,
in Gegenwart eines Palladium-Katalysators und einer Base bei Temperaturen von 30 bis 160°C umsetzt, die erhaltenen Verbindungen in Gegenwart einer Base in Verbindungen der Formel 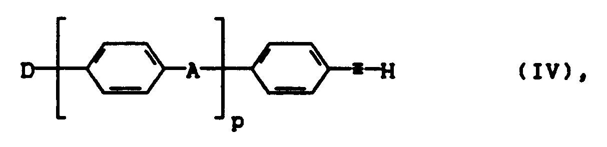
worin
- D, A und p: die obengenannte Bedeutung haben,
überführt, danach die Verbindungen der Formel (IV) mit Verbindungen der Formel 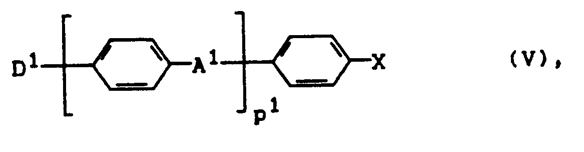
worin
- D¹, A¹ und p¹: die für A, D und p beschriebene Bedeutung besitzen und
- X: für Brom oder Iod steht,
in Gegenwart eines Palladium-Katalysators und einer Base bei Temperaturen von 30 bis 160°C umsetzt, anschließend die erhaltenen Acetylen-Verbindungen in Gegenwart von Katalysatoren mit Wasserstoff oder mit anderen Reduktionsmitteln zu Z-Stilbenverbindungen der Formel 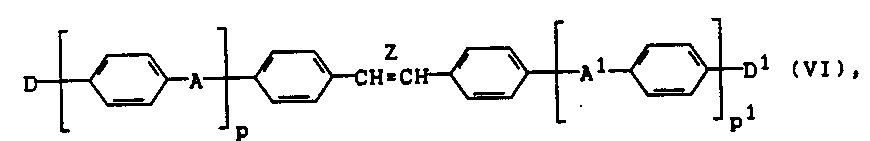
worin
- D, D¹, A, A¹ und p, p¹: die obengenannte Bedeutung haben,
reduziert und die funktionellen Gruppen D und D¹ in die obengenannten funktionellen Gruppen Z und Y der Formel (I) in bekannter Weise überführt
oder
die Z-Stilbenverbindungen der Formel (VI) im Fall, daß D und D¹ mindestens für ein Bromatom stehen mit Verbindungen der Formel

H₂C=CHR⁷ (VII),

worin
- R⁷: für Wasserstoff oder 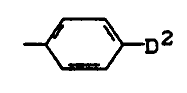 steht, wobei
- D²: die für D¹ genannte Bedeutung besitzt,
in Gegenwart eines Palladium-Katalysators und einer Base bei Temperaturen von 30 bis 160°C umsetzt und die funktionellen Gruppen D, D¹ und D² in die funktionellen Gruppen Z und Y der Formel (I) in üblicher Weise überführt
oder
die Verbindungen der Formel (IV) mit Verbindungen der Formel 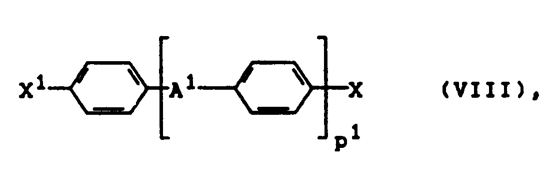
worin
- A¹, X und p¹: die zuvor genannte Bedeutung besitzen und
- X¹: für Brom oder Iod steht,
in Gegenwart eines Palladium-Katalysators und einer Base bei Temperaturen von 30 bis 160°C umsetzt, anschließend die erhaltenen Acetylenverbindungen in Gegenwart von Katalysatoren mit Wasserstoff oder mit anderen Reduktionsmitteln zu entsprechenden Z-Stilbenverbindungen reduziert und danach die funktionellen Gruppen D in die funktionellen Gruppen Z und Y der Formel (I) in bekannter Weise überführt.

Die Umsetzung der aromatischen Halogenide der allgemeinen Formel (II) mit den Acetylenen der Formel (III) kann nach einer im Prinzip bekannten Methode (siehe z.B. A. Segnitz in Houben-Weyl, Bd. 13/9b, S. 987 ff) durchgeführt werden. Danach wird die Umsetzung in Gegenwart eines Palladium-Katalysators und einer basisch reagierenden Verbindung, gegebenenfalls in Gegenwart eines Co-Katalysators, eines Phosphans und eines Lösungs- und/oder Verdünnungsmittels bei Temperaturen von ca. 30 bis 160°C, bevorzugt 40 bis 130°C, durchgeführt.

Bei dieser Umsetzung entstehen Verbindungen der allgemeinen Formel (IX) 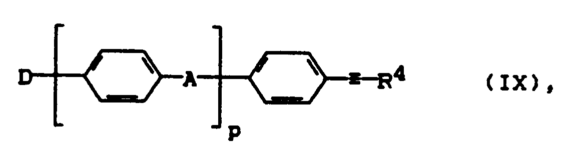
in der
- D, A, p und R⁴: die zuvor genannte Bedeutung besitzen.

Bevorzugte aromatische Halogenide der Formel (II) sind beispielsweise solche, in denen A für eine chemische Bindung oder CH=CH, X für Brom oder Iod, p für 0 oder 1 und D für COOR¹, COR¹, NHR², NO₂, OR³, Cl oder Br stehen mit R¹ = Wasserstoff oder verzweigtes oder geradkettiges C₁-C₈-Alkyl, R² = OAc, COOC₂H₅ oder eine andere geeignete Schutzgruppe und R³ = OAc, 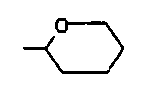
oder eine andere geeignete Schutzgruppe.

Genannt werden beispielsweise als aromatische Halogenide der Formel (II): 4-Brombenzoesäure, 4-Brombenzoesäuremethylester, 4-Brombenzoesäureethylester, 4-Brombenzoesäure-n-propylester, 4-Brombenzoesäure-i-propylester, 4-Brombenzoesäure-n-butylester, 4-Brombenzoesäure-i-butylester, 4-Brombenzoesäure-tert.-butylester, 4-Brombenzoesäure-n-pentylester, 4-Bromnitrobenzol, 4-Brombenzaldehyd, 4-Acetoxybrombenzol, 4-Acetoxyiodbenzol, 4-Bromphenyltetrahydropyranylether, 4-Iodphenyltetrahydropyranylether, 1,4-Dibrombenzol, 1-Brom-4-iodbenzol, 4-Bromacetophenon, (4-Bromphenyl)ethylketon, (4-Bromphenyl)-n-propylketon, (4-Bromphenyl)-i-propylketon, (4-Bromphenyl)-n-butylketon, 4-Bromacetanilid, 4-Iodacetanilid, 4-Bromphenylcarbamidsäureethylester, 1-Brom-4-chlorbenzol, 1-Chlor-4-iodbenzol, 4-Brombiphenyl-4′-carbonsäuremethylester, 4-Brombiphenyl-4′-carbonsäureethylester, 4-Brombiphenyl-4′-carbonsäure-n-propylester, 4-Brombiphenyl-4′-carbonsäure-i-propylester, 4-Brombiphenyl-4′-carbonsäure-n-butylester, 4-Brombiphenyl-4′-carbonsäure-i-butylester, 4-Brombiphenyl-4′-carbonsäure-tert.-butylester, 4-Brombiphenyl-4′-carbonsäure-n-pentylester, 4-Brom-4′-nitrobiphenyl, 4-Acetoxy-4′-brombiphenyl, 4-Brombiphenyl-4′-tetrahydropyranylether, 4-Brom-4′-iodbiphenyl, 4,4′-Dibrombiphenyl, 4-Brom-4′-chlorbiphenyl, (4-Brombiphenyl-4′-yl)-methylketon, (4-Brombiphenyl-4′-yl)ethylketon, (4-Brombiphenyl-4′-yl)-n-propylketon, (4-Brombiphenyl-4′-yl)-i-propylketon, (4-Brombiphenyl-4′-yl)-n-butylketon, 4-Acetamino-4′-brombiphenyl, 4-Brombiphenyl-4′-carbamidsäureethylester, 4-Bromstilben-4′-carbonsäuremethylester, 4-Bromstilben-4′-carbonsäureethylester, 4-Bromstilben-4′-carbonsäure-n-propylester, 4-Bromstilben-4′-carbonsäure-n-butylester, 4-Bromstilben-4′-carbonsäure-i-propylester, 4-Bromstilben-4′-carbonsäure-i-butylester, 4-Bromstilben-4′-carbonsäure-tert.-butylester, 4-Bromstilben-4′-carbonsäure-n-pentylester, 4-Brom-4′-nitrostilben, 4-Acetoxy-4′-bromstilben, 4-Bromstilben-4′-tetrahydropyranylether, 4-Brom-4′-iodstilben, 4,4′-Dibromstilben, 4-Brom-4′-chlorstilben, (4-Bromstilbenyl-4′)-methylketon, (4-Bromstilbenyl-4′)-ethylketon, (4-Bromstilbenyl-4′)-n-propylketon, (4-Bromstilbenyl-4′)-i-propylketon, (4-Bromstilbenyl-4′)-n-butylketon, 4-Acetamino-4′-bromstilben und 4-Bromstilben-4′-carbamidsäureethylester, bevorzugt 4-Brombenzoesäure, 4-Brombenzoesäuremethylester, 4-Brombenzoesäureethylester, 4-Brombenzoesäure-n-propylester, 4-Brombenzoesäure-i-propylester, 4-Brombenzoesäure-n-butylester, 4-Brombenzoesäure-i-butylester, 4-Brombenzoesäure-tert.-butylester, 4-Bromnitrobenzol, 4-Brombenzaldehyd, 4-Acetoxybrombenzol, 4-Acetoxyiodbenzol, 4-Bromphenyltetrahydropyranylether, 4-Iodphenyltetrahydropyranylether, 1,4-Dibrombenzol, 1-Brom-4-iodbenzol, 4-Bromacetophenon, (4-Bromphenyl)-ethylketon, (4-Bromphenyl)-n-propylketon, 4-Bromacetanilid, 4-Iodacetanilid, 4-Bromphenylcarbamidsäureethylester, 1-Brom-4-chlorbenzol, 4-Brombiphenyl-4′-carbonsäuremethylester, 4-Brombiphenyl-4′-carbonsäureethylester, 4-Brombiphenyl-4′-carbonsäure-n-propylester, 4-Brombiphenyl-4′-carbonsäure-i-propylester, 4-Brombiphenyl-4′-carbonsäure-n-butylester, 4-Brombiphenyl-4′-carbonsäure-i-butylester, 4-Brombiphenyl-4′-carbonsäure-tert.-butylester, 4-Brom-4′-nitrobiphenyl, 4-Acetoxy-4′-brombiphenyl, 4-Brom-4′-iodbiphenyl, 4,4′-Dibrombiphenyl, 4-Brom-4′-chlorbiphenyl, (4-Brombiphenyl-4′-yl)-methylketon, (4-Brombiphenyl-4′-yl)-ethylketon, (4-Brombiphenyl-4′-yl)-n-propylketon, 4-Brombiphenyl-4′-carbamidsäureethylester, 4-Bromstilben-4′-carbonsäuremethylester, 4-Bromstilben-4′-carbonsäureethylester, 4-Bromstilben-4′-carbonsäure-n-propylester, 4-Bromstilben-4′-carbonsäure-n-butylester, 4-Bromstilben-4′-carbonsäure-i-propylester, 4-Bromstilben-4′-carbonsäure-i-butylester, 4-Bromstilben-4′-carbonsäure-tert.-butylester, 4-Brom-4′-nitrostilben, 4-Acetoxy-4′-bromstilben, 4-Bromstilben-4′-tetrahydropyranylether, 4-Brom-4′-iodstilben, 4,4′-Dibromstilben, 4-Brom-4′-chlorstilben, (4-Bromstilbenyl-4′)-methylketon, (4-Bromstilbenyl-4′)-ethylketon, (4-Bromstilbenyl-4′)-n-propylketon und 4-Bromstilben-4′-carbamidsäureethylester, besonders bevorzugt 4-Brombenzoesäure, 4-Brombenzoesäuremethylester, 4-Brombenzoesäureethylester, 4-Brombenzoesäure-n-butylester, 4-Brombenzoesäure-tert.-butylester, 4-Bromnitrobenzol, 4-Acetoxybromphenol, 4-Bromphenyltetrahydropyranylether, 4-Iodphenyltetrahydropyranylether, 1,4-Dibrombenzol, 1-Brom-4-iodbenzol, 4-Bromacetophenon, (4-Bromphenyl)ethylketon, 4-Bromacetanilid, 4-Bromphenylcarbamidsäureethylester, 1-Brom-4-chlorbenzol, 4-Brombiphenyl-4′-carbonsäuremethylester, 4-Brombiphenyl-4′-carbonsäureethylester, 4-Brombiphenyl-4-carbonsäure-n-butylester, 4-Brombiphenyl-4′-carbonsäure-tert.-butylester, 4-Brom-4′-nitrobiphenyl, 4-Acetoxy-4′-brombiphenyl, 4-Brom-4′-iodbiphenyl, 4,4′-Dibrombiphenyl, 4-Brom-4′-chlorbiphenyl, (4-Brombiphenyl-4′-yl)-methylketon, (4-Brombiphenyl-4′-yl)-ethylketon, (4-Brombiphenyl-4′-yl)-n-propylketon, 4-Brombiphenyl-4′-carbamidsäureethylester, 4-Bromstilben-4′-carbonsäureethylester, 4-Bromstilben-4-carbonsäure-n-propylester, 4-Bromstilben-4′-carbonsäuretert.-butylester, 4-Brom-4′-nitrostilben, 4-Acetoxy-4′-bromstilben, 4-Bromstilben-4′-tetrahydropyranylether, 4-Brom-4′-iodstilben, 4,4′--Dibromstilben, 4-Brom-4′-chlorstilben, (4-Bromstilbenyl-4′)-methylketon, (4-Bromstilbenyl-4′)-ethylketon, (4-Bromstilbenyl-4′)-n-propylketon und 4-Bromstilben-4′-carbamidsäureethylester.

Bevorzugte Verbindungen der Formel (III) sind solche, worin R⁵ und R⁶ für Methyl oder Ethyl stehen. Genannt werden 2-Methyl-3-butin-2-ol und Trimethylsilylacetylen.

Nach dem erfindungsgemäßen Verfahren setzt man pro Mol der Verbindung der Formel (II) ca. 0,8 bis 1,2 Mol, vorzugsweise 1,0 bis 1,2 Mol der Verbindung (III) ein. Geeignete Palladium-Katalysatoren sind z.B. beschrieben in R.F. Heck, Palladium Reagents in Organic Synthesis, Academic Press, New York 1985, Chapter 6, Section 6.8.1). In Frage kommen z.B. Pd(OAc)₂, PdCl₂(Pφ₃)₂, Pd(OAc)₂(Pφ₃)₂, Pd(Pφ₃)₄ und/oder PdCl₂, die in einer Menge von ca. 0,005-10 Mol-%, bevorzugt 0,05-5 Mol-%, bezogen auf das Arylhalogenid der Formel (II), eingesetzt werden (φ bedeutet Phenyl).

Um den während der Reaktion freiwerdenden Halogenwasserstoff zu binden wird die basisch reagierende Verbindung gegebenenfalls auch im Überschuß eingesetzt. Die basisch reagierende Verbindung kann gegebenenfalls auch als Verdünnungsmittel dienen. Geeignete, basisch reagierende Verbindungen sind z.B. sekundäre und/oder tertiäre Amine, wie Diethylamin, Diisopropylamin, Piperidin, Triethylamin, Pyridin und N,N′-Diethylanilin, Salze von Carbonsäuren, wie Kaliumacetat, Alkalicarbonate wie Kaliumcarbonat, Alkalibicarbonate, wie Natriumbicarbonat, Alkalialkoholate, wie Natriummethanolat und Natriumethanolat und Alkalihydride, wie Natriumhydrid.

Als Co-Katalysator kann in die Umsetzung der Verbindung (II) mit der Verbindung (III) beispielsweise Kupfer(I)-iodid in einer Menge von ca. 10 bis 1000 Mol-%, bezogen auf eingesetztes Palladium, zugesetzt werden. Darüber hinaus kann die Katalysatorwirksamkeit durch Zusatz von Phosphanen verbessert werden. Als bevorzugt einzusetzende Phosphane sind z.B. Triphenylphosphan und/oder Tri-o-tolylphosphan zu nennen. Die Menge der einzusetzenden Phosphane kann leicht durch Vorversuche ermittelt werden. Üblicherweise werden die Phosphane eingesetzt in einer Menge von ca. 100 bis 1000 Mol-%, bezogen auf eingesetztes Palladium.

Unter Umständen ist es vorteilhaft, die erfindungsgemäße Umsetzung der Verbindungen der Formel (II) mit denen der Formel (III) unter Zusatz eines Lösungs- und/oder Verdünnungsmittels durchzuführen. Als Lösungs- und/oder Verdünnungsmittel kommen z.B. in Frage: aromatische Kohlenwasserstoffe, wie Benzol, Toluol, Xylol, Ether, wie Tetrahydrofuran, Dioxan, Alkohole, wie Methanol oder Ethanol, ferner Acetonitril, Dimethylsulfoxid, Dimethylformamid oder N-Methyl-pyrrolidon, sowie Amine, wie Diethylamin oder Triethylamin. Die Lösungs- und/oder Verdünnungsmittel können sowohl einzeln als auch im Gemisch untereinander eingesetzt werden. Die Menge kann in weiten Bereichen variiert und gegebenenfalls durch Vorversuche leicht ermittelt werden.

Weiterhin ist es unter Umständen günstig, die Umsetzung in einer Inertgasatmosphäre, z.B. unter einer Stickstoff-Atmosphäre, durchzuführen. Dies hängt z.B. von den eingesetzten Ausgangsprodukten ab.

Die bei der Umsetzung der Verbindungen der Formel (II) mit den Verbindungen der Formel (III) erhaltenen Verbindungen der Formel (IX) werden durch Behandlung mit basisch reagierenden Verbindungen, gegebenenfalls in geeigneten Lösungs- und/oder Verdünnungsmitteln, zu Acetylenen der allgemeinen Formel (IV) umgesetzt. Dabei wird der Rest R⁴ der Verbindungen der Formel (IX) gegen Wasserstoff ausgetauscht. Solche Methoden sind z.B. beschrieben von V. Jäger in Houben-Weyl, Bd. V/2a, S. 405 und S. 649.

Als geeignete, basisch reagierende Verbindungen sind z.B. Alkalihydroxide, wie Natriumhydroxid und/oder Kaliumhydroxid, Alkalicarbonate, wie Natriumcarbonat und/oder Kaliumcarbonat sowie Alkalihydride, wie Natriumhydrid, zu nennen. Die Menge der einzusetzenden basisch reagierenden Verbindungen läßt sich leicht durch Vorversuche ermitteln und beträgt üblicherweise 1 bis 100 Mol-%, bevorzugt 3 bis 50 Mol-%, bezogen auf die eingesetzte Verbindung (IX).

Als Lösungs- und/oder Verdünnungsmittel kommen bei der beschriebenen Reaktion z.B. in Frage: aromatische Kohlenwasserstoffe, wie Benzol, Toluol, Xylol, oder Alkohole, wie Methanol oder Ethanol, sowie Gemische der genannten Lösungs- und/oder Verdünnungsmittel.

Die Reaktionstemperaturen bei der genannten Reaktion hängen stark vom betrachteten Einzelfall ab. Wird in einem Lösungs-und/oder Verdünnungsmittel gearbeitet, so wählt man im allgemeinen Temperaturen, die bei -20°C bis zum Siedepunkt des eingesetzten Lösungs-und/oder Verdünnungsmittels bzw. deren Gemische liegen.

Wenn in der Formel (IX) R⁴ für C(CH₃)₂-OH steht, so kann das bei der beschriebenen Reaktion gebildete Aceton gegebenenfalls kontinuierlich abdestilliert werden. Steht R⁴ in der Formel (IX) für Si(CH₃)₃, kann die Schutzgruppe auch durch Behandlung mit Kaliumfluoriddihydrat oder Kaliumfluorid in einem geeigneten Lösungs-und/oder Verdünnungsmittel, wie Methanol, Ethanol und/oder Dimethylformamid, gegen Wasserstoff ausgetauscht werden.

Nach dem erfindungsgemäßen Verfahren werden die Verbindungen der Formel (IV) anschließend in Gegenwart eines Palladium-Katalysators und einer basisch reagierenden Verbindung, gegebenenfalls in Gegenwart eines Co-Katalysators, gegebenenfalls in Gegenwart eines Phosphans und gegebenenfalls eines Lösungs- und/oder Verdünnungsmittels bei Temperaturen von 30 bis 160°C, bevorzugt bei 40 bis 130°C, gegebenenfalls in einer Inertgasatmosphäre umgesetzt mit Verbindungen der Formel (V). Dabei entstehen Diarylacetylene der Formel (X) 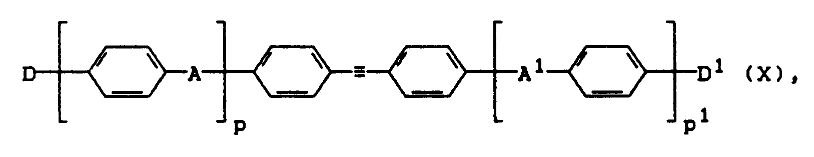
worin
D, D¹, A, A¹, p, p¹ die oben angegebenen Bedeutungen haben.

Bei der Umsetzung der Verbindungen der Formel (IV) mit den aromatischen Halogeniden der Formel (V) zu den Diarylacetylenen der Formel (X) setzt man im allgemeinen pro Mol der Verbindung (V) 0,7 bis 1,3 Mol, bevorzugt 0,8 bis 1,2 Mol der Verbindung der Formel (IV) ein.

Nach dem erfindungsgemäßen Verfahren können die Verbindungen der Formel (IV) auch mit aromatischen Dihalogeniden der allgemeinen Formel (VIII) 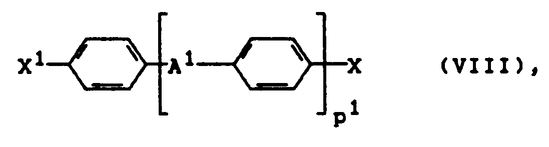
worin
X¹ und X sowie A¹ und p¹ die zuvor genannte Bedeutung besitzen,
zu Diacetylenverbindungen der Formel (XI) 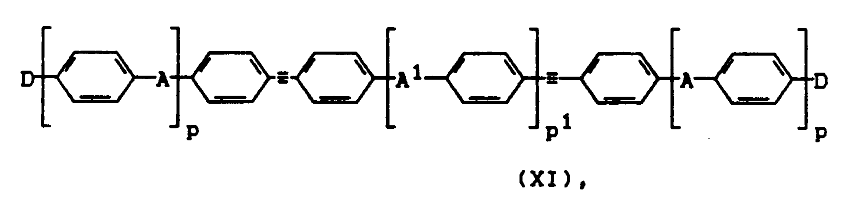
wobei
D, A, A¹, p, p¹ die oben angegebene Bedeutung haben,
umgesetzt werden.

In diesem Fall setzt man im allgemeinen pro Mol der Verbindung der Formel (VIII) ca. 2,0 bis 2,6 Mol, bevorzugt 2,0 bis 2,2 Mol, der Verbindung der Formel (IV) ein.

Die Umsetzung wird wiederum, wie zuvor beschrieben, in Gegenwart eines Palladium-Katalysators und einer basisch reagierenden Verbindung bei Temperaturen im Bereich von 30 bis 160°C durchgeführt.

Bevorzugte Verbindungen der Formel (VIII) sind z.B. 1,4-Diiodbenzol, 1-Brom-4-iodbenzol, 1,4-Dibrombenzol, 4,4′-Diiodbiphenyl, 4-Brom-4′-iodbiphenyl, 4,4′-Dibrombiphenyl, 4,4′-Diiodstilben, 4-Brom-4′-iodstilben und 4,4′-Dibromstilben, besonders bevorzugt 1,4-Dibrombenzol, 4,4′-Dibrombiphenyl und 4,4′-Dibromstilben.

Hinsichtlich Palladium-Katalysator, Base, Co-Katalysator, Phosphanzusatz und Verdünnungs- bzw. Lösungsmittel gilt das gleiche wie bei der Umsetzung der Verbindungen der Formel (II) mit denen der Formel (III) beschrieben.

Die bei der Umsetzung erhaltenen Acetylene der Formel (X) sowie die erhaltenen Diacetylene der Formel (XI) werden anschließend in Gegenwart von Katalysatoren mit Wasserstoff oder mit anderen geeigneten Reduktionsmitteln zu den entsprechenden Z-Stilbenverbindungen reduziert; und zwar zu Z-Stilbenverbindungen der Formel (VI) 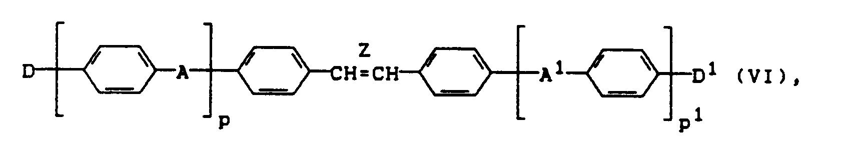
worin
D, A, A¹, p, p¹ die zuvor genannte Bedeutung haben,
und zu Z-Stilbenverbindungen der Formel (XII) 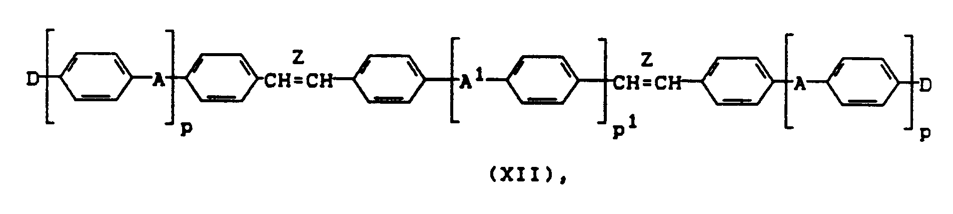
worin
D, A, A¹, p, p¹ die obengenannte Bedeutung haben.

Geeignete Reduktionsverfahren sind z.B. die Hydrierung in Gegenwart von Hydrierkatalysatoren, wie speziellen Palladium- oder Nickel-Katalysatoren. Solche Katalysatoren sind beispielsweise beschrieben von H. Balli in Houben-Weyl, Bd. V/1b, S. 588 ff; H. Gutmann, H. Lindlar in Chemistry of Acetylenes, H.G. Viehe Ed., Marcel Dekker, New York 1969, S. 355 ff; J.J. Brunet, P. Caubere, J. Org. Chem. 49, 4058 (1984), die Reduktion mit Systemen aus Metall und protonenabspaltendem Mittel, wie die Reduktion mit Zink oder Zink/Kupfer-Paaren in Essigsäuren, Alkoholen oder Wasser (beschrieben z.B. von H. Balli in Houben-Weyl, Bd. V/1b, S. 583; B.L. Sondengam, G. Charles, T.H. Akam, Tetrahedron Lett. 1069 (1980); M.H.P.J. Aerssens, L. Brandsma, J. Chem. Soc. Chem. Commun. 735 (1984), die Überführung von Acetylenen in Vinylsilane und anschließenden stereoselektiven Austausch der Silylgruppe gegen Wasserstoff (siehe z.B. D.G. Batt, B. Ganem, Tetrahedron Lett. 3323 (1978)) sowie die Monohydroborierung von Acetylenen und anschließende Protolyse, die Reduktion mit Dialkylaluminiumhydriden oder die Reduktion mit Diimin (s. H. Balli in Houben-Weyl, Bd. V/1b, S. 583 ff).

Nach dem erfindungsgemäßen Verfahren können die Verbindungen der Formel (VI) im Fall, daß D und D¹ mindestens für ein Bromatom steht, mit Verbindungen der Formel (VII) in Gegenwart eines Palladium-Katalysators und einer basisch reagierenden Verbindung, gegebenenfalls in Gegenwart eines Co-Katalysators, gegebenenfalls in Gegenwart eines Phosphans und gegebenenfalls in Gegenwart eines Lösungs- und/oder Verdünnungsmittels bei Temperaturen von 30 bis 160°C, bevorzugt bei 40 bis 130°C, gegebenenfalls unter Inertgasatmosphäre, zu Verbindungen der allgemeinen Formeln (XIII), (XIV) und (XV) mit den zuvor genannten Bedeutungen für D, A, p, A¹, p¹ und D¹ und D² umgesetzt werden:

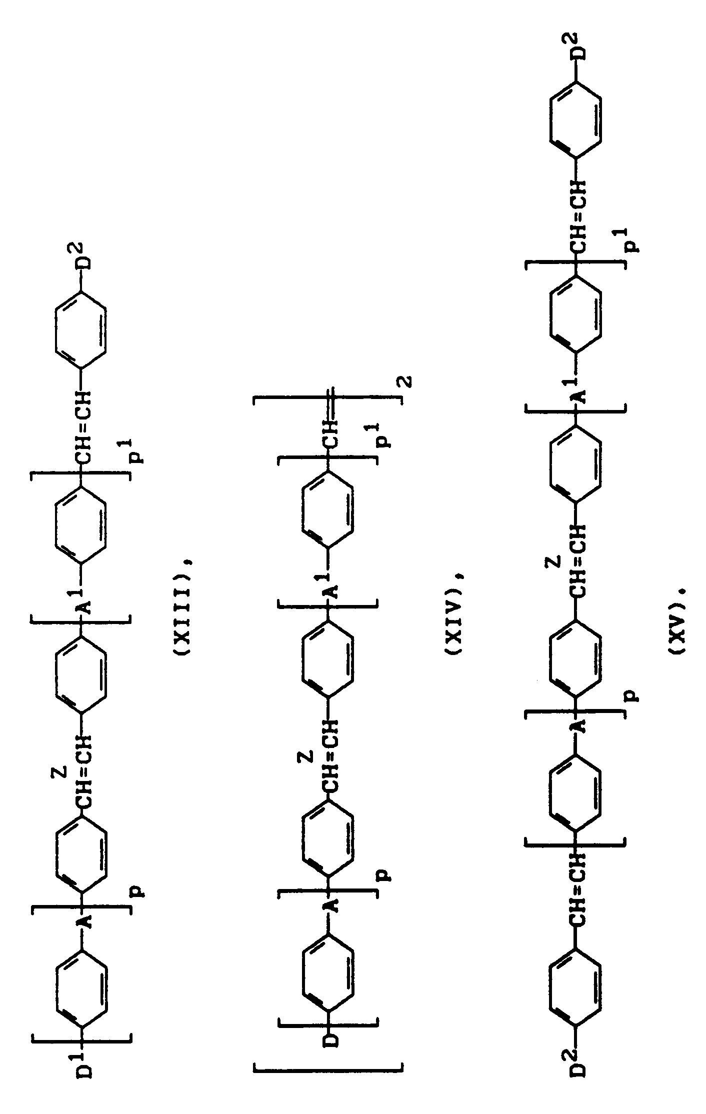

Die Umsetzung aromatischer Halogenide, insbesondere aromatischer Bromide und Iodide, mit Ethylen oder Ethylenderivaten ist eine an sich bekannte Reaktion (siehe z.B. R.F. Heck, Org. React. 27, 345 (1982)) und wird hier in allgemein üblicher Weise durchgeführt.

Die Verbindungen der Formel (VII) sind allgemein bekannte Verbindungen der organischen Chemie oder lassen sich leicht nach analogen Verfahren herstellen (siehe z.B. W. Heitz et al., Makromol. Chem. 189, 119 (1988)).

Bevorzugte Verbindungen der Formel (VII) sind z.B.: 4-Carboxystyrol, 4-Carbomethoxystyrol, 4-Carboethoxystyrol, 4-Carbo-n-propoxystyrol, 4-Carbo-i-propoxystyrol, 4-Carbo-n-butoxystyrol, 4-Carbo-i-butoxystyrol, 4-Carbo-tert.-butoxystyrol, 4-Acetaminostyrol, 4-Acetoxystyrol, 4-Nitrostyrol, (Styryl-4)methylketon, (Styryl-4)ethylketon, (Styryl-4)-n-propylketon, (Styryl-4)-i-propylketon, (Styryl-4)-n-butylketon, 4-Chlorstyrol und 4-Aminostyrol.

Die so erhaltenen Z-Stilbenverbindungen mit den funktionellen Gruppen D, D¹ und D² werden dann zum Abschluß des erfindungsgemäßen Verfahrens durch im Prinzip bekannte Methoden in die beanspruchten, bifunktionellen, mindestens eine Z-konfigurierte Stilbengruppierung enthaltenden Stilbenverbindungen der Formel (I) mit den funktionellen Gruppen Y und Z überführt.

Die Überführung der funktionellen Gruppen kann beispielsweise durchgeführt werden durch alkalische oder saure Hydrolyse von Estern zu den entsprechenden Säuren, die protonenkatalysierte Spaltung von tert.-Butylestern in freie Carbonsäuren und Isobuten, die Überführung von Carbonsäuren in ihre Chloride durch Umsetzung mit z.B. Thionylchlorid oder Oxalylchlorid, gegebenenfalls in Gegenwart katalytischer Mengen DMF oder Pyridin, ferner die Oxidation von Arylketonen zu den entsprechenden Arylcarbonsäuren, die Reduktion von Nitro- zu Aminogruppen, die Überführung von Amino- oder Carboxyl- in Isocyanatgruppen oder auch die Umsetzung von Hydroxylgruppen in die entsprechenden Chlorkohlensäureester. Diese Methoden sind allgemein bekannt und z.B. beschrieben in "Organikum", Autorenkollektiv, VEB Deutscher Verlag der Wissenschaften, Berlin 1976; J. March, Advanced Organic Chemistry, Wiley, 3rd Ed. 1985; C. Ferri, Reaktionen der organischen Synthese, Stuttgart, Thieme 1978.

Das erfindungsgemäße Verfahren läßt sich durch das nachfolgende Formelschema darstellen, das beispielhaft die Synthese von Z-1-(4-Carboxyphenyl)-2-(4′-carboxybiphenyl-4-yl)ethen beschreibt: 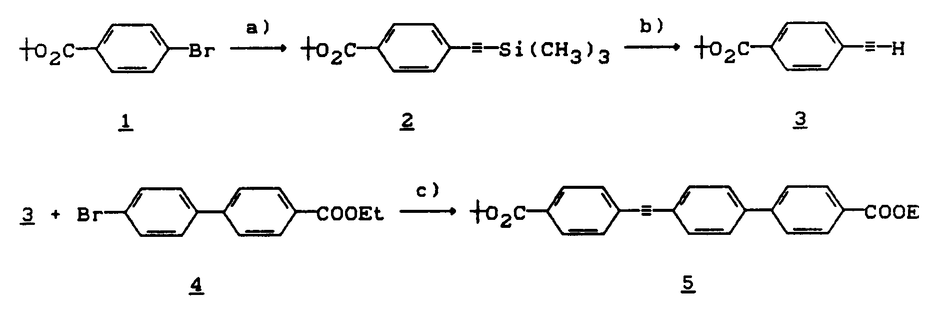 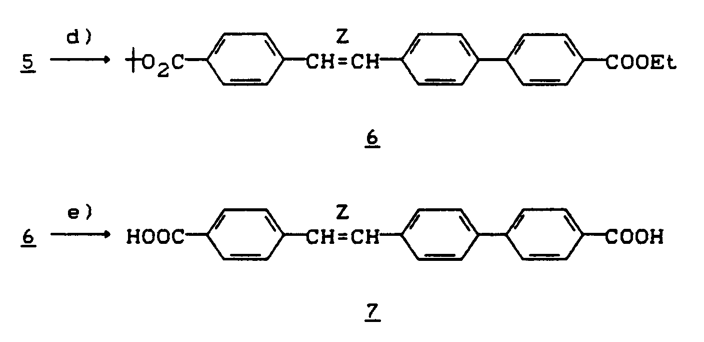
a) H-≡-Si(CH₃)₃, (φ₃P)₂PdCl₂, CuI, φ₃P, CH₃CN, NEt₃
b) K₂CO₃, CH₃OH
c) (φ₃P)₂PdCl₂, CuI, φ₃P, CH₃CN, NEt₃
d) Zn/Cu, C₂H₅OH, THF
e) 1. p-TsOH, Toluol, 2. NaOH, H₂O, C₂H₅OH 3. HCl

Nach einer Variante des erfindungsgemäßen Verfahrens ist es möglich, Arylhalogenide der Formel (II) mit Acetylenen der Formel (III) in Gegenwart eines Palladium-Katalysators und einer Base, gegebenenfalls in Gegenwart eines Co-Katalysators, eines Phosphans, eines Lösungsund/oder Verdünnungsmittels und eines Phasentransferkatalysators bei Temperaturen von 40°C bis 170°C, bevorzugt bei 60°C bis 130°C, zu den entsprechenden Diarylacetylenen der Formel (X) direkt umzusetzen, wobei in der Formel (X) D = D¹, A = A¹ und p = p¹ ist und die für die Formel (X) angegebene Bedeutung besitzen.

Als Arylhalogenide der Formel (II) werden bevorzugt solche eingesetzt, wie sie bei der vorhergehenden Aufzählung der Arylhalogenide der Formel (II) genannt wurden.

Als Acetylenverbindung der Formel (III) wird bevorzugt 2-Methyl-3-butin-2-ol eingesetzt.

Bei dieser Variante werden auf 2 Mol des Arylhalogenids der Formel (II) im allgemeinen 0,8 bis 1,2 Mol, vorzugsweise 0,9 bis 1,1 Mol, des Acetylens der Formel (III) eingesetzt. Als Palladium-Katalysatoren werden die bereits zuvor genannten verwendet.

Die Umsetzung gemäß dieser Variante wird in Gegenwart einer basisch reagierenden Verbindung durchgeführt. Geeignete basisch reagierende Verbindungen sind beispielsweise Alkalihydroxide, wie Natriumhydroxid und Kaliumhydroxid, Alkalialkoholate, wie Natriumethanolat und Natriummethanolat, Alkalihydride, wie Natriumhydrid, Alkaliamide, wie Natriumamid, Alkalicarbonate, wie Natriumcarbonat und Kaliumcarbonat, Alkalihydrogencarbonate, wie Natriumhydrogencarbonat und Kaliumhydrogencarbonat, sowie Gemische der basisch reagierenden Verbindungen untereinander. Es ist auch möglich, die Umsetzung gemäß dieser Variante in Gegenwart einer wäßrigen Lösung der zuvor genannten basisch reagierenden Verbindungen durchzuführen. Außerdem ist es möglich, zur Beschleunigung der Umsetzung einen Co-Katalysator mitzuverwenden. Als Co-Katalysatoren kommen die bereits zuvor genannten in Frage. Ebenfalls mitverwendet werden können bei der Umsetzung gemäß dieser Variante Phosphane. Die in Frage kommenden Phosphane wurden bereits zuvor beschrieben.

Falls es für die Umsetzung förderlich ist, kann in Gegenwarteines Lösungs- und/oder Verdünnungsmittels gearbeitet werden. Geeignete Lösungs- und/oder Verdünnungsmittel sind z.B. aromatische Kohlenwasserstoffe, wie Benzol, Toluol und/oder Xylol, Amide, wie Dimethylformamid und/oder NMP und/oder Sulfoxide, wie Dimethylsulfoxid oder Alkohole, wie Methanol und/oder Ethanol.

Der Zusatz eines Phasentransferkatalysators bei der Umsetzung der Verbindungen der Formel (II) mit Verbindungen der Formel (III) gemäß dieser Variante ist ebenfalls möglich. Geeignete Phasentransferkatalysatoren sind z.B. beschrieben in E.V. Dehmlow, S.S. Dehmlow, Phase Transfer Catalysis, 2d Ed., Verlag Chemie, Deerfield Beach, Fla., 1983. Genannt seien: Tetraethylammoniumchloridmonohydrat, Tetraethylammoniumbromid, Tetraethylammoniumiodid, Tetraethylammoniumtetrafluoroborat, Tetraethylammonium-p-toluolensulfonat, Allyltriethylammoniumbromid, n-Hexyl-trimethylammoniumbromid, Phenyltriethylammoniumchlorid, Phenyltrimethylammoniumiodid, Benzyltrimethylammoniumbromid, Benzyltrimethylammoniumiodid, n-Octyltrimethylammoniumbromid, Tetra-n-propylammoniumbromid, Tetra-n-propylammoniumhydrogensulfat, Tetra-n-propylammoniumtrifluoromethansulfonat. Benzyltriethylammoniumchlorid, Benzyltriethylammoniumbromid, Benzyltriethylammoniumtetrafluoroborat, n-Dodecyltrimethylammoniumbromid, Tetra-n-butylammoniumchlorid, Tetra-n-butylammoniumbromid, Tetra-n-butylammoniumiodid, Tetra-n-butylammoniumhydrogensulfat, Tetra-n-butylphosphoniumbromid, Tetraphenylphosphoniumchlorid, Tetraphenylphosphoniumbromid, Tetraphenylphosphoniumiodid, Tetraphenylphosphoniumhexafluoroantimonat, Tetraphenylphosphaniumtetrafluoroborat, N-Hexadecylpyridiniumbromid, Tetra-n-hexylammoniumbromid, Tetra-n-hexylammoniumhydrogensulfat, n-Hexadecyl-tri-n-butylphosphoniumbromid, Triphenylmethyl-triphenylphosphoniumchlorid, Tetra-n-octylammoniumbromid sowie Tetra-n-dodecylammoniumiodid.

Es kann weiterhin vorteilhaft sein, die Umsetzung in einer Inertgasatmosphäre, z.B. N₂-Atmosphäre, durchzuführen.

Im allgemeinen wird die Umsetzung gemäß dieser Variante so durchgeführt, daß man, gegebenenfalls in einem geeigneten Lösungs- und/oder Verdünnungsmittel, das Arylhalogenid der Formel (II), das Acetylen der Formel (III), den Palladium-Katalysator, gegebenenfalls den Co-Katalysator, das Phosphan und den Phasentransferkatalysator vorlegt, mit der basisch reagierenden Verbindung versetzt und das Reaktionsgemisch auf eine für die Umsetzung ausreichende Temperatur erwärmt. Es ist natürlich auch möglich, die Reaktionskomponenten in einer anderen Reihenfolge zusammenzugeben. Das Acetylen der Formel (III) kann auch, gegebenenfalls gelöst in einem der obengenannten Lösungs- und/oder Verdünnungsmittel, bei Reaktionstemperatur innerhalb eines angemessenen Zeitraums, d.h. innerhalb von ca. 0,5 bis 24 Stunden, zu den anderen Reaktionskomponenten zugetropft werden. Das Fortschreiten der Reaktion kann beispielsweise gaschromatographisch verfolgt werden. Zur Aufarbeitung werden im allgemeinen die Phasen getrennt und das Reaktionsprodukt unter Anwendung üblicher Methoden isoliert. Unter Umständen fällt das Reaktionsprodukt schon während der Reaktion oder beim Abkühlen nach beendeter Reaktion aus, in diesem Fall wird abgesaugt und zusätzlich die flüssigen Phasen mit üblichen Methoden aufgearbeitet. Das Reaktionsprodukt kann gegebenenfalls durch Kristallisation, Destillation oder Chromatographie gereinigt werden.

Der Reaktionsablauf des Verfahrens nach dieser Variante läßt sich durch das folgende Formelschema darstellen, das beispielhaft die Synthese von Z-4,4′-Stilbendicarbansäuredichlorid beschreibt: 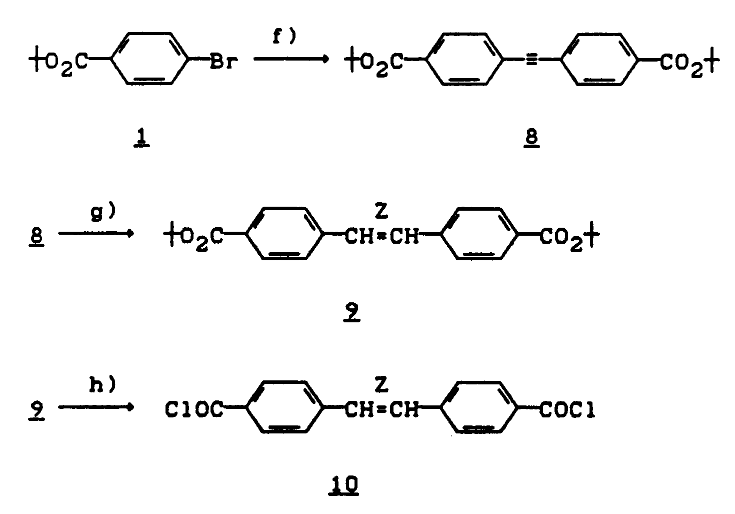
f) ≡┼OH, (φ₃P)₂PdCl₂, CuI, Pφ₃, NaOH, H₂O, Toluol, BzNEt₃Cl
g) Zn/Cu, EtOH/THF
h) 1. p-TsOH, Toluol 2. NaOH, H₂O, EtOH 3. HCl 4. SOCl₂. DMF

Die Schritte h) 2. und 3. dienen hier lediglich einer weiteren Reinigung des Reaktionsproduktes.

Gemäß einer weiteren Variante können Verbindungen der Formel (X) für die D ≠ D¹ und/oder A ≠ A¹ und/oder p ≠ p¹ gilt, durch Umsetzung der Verbindungen der allgemeinen Formel (IX) mit Arylhalogeniden der allgemeinen Formel (V) in Gegenwart eines Palladium-Katalysators und einer basisch reagierenden Verbindung, gegebenenfalls in Gegenwart eines Co-Katalysators, eines Phosphans, eines Lösungs- und/oder Verdünnungsmittels und eines Phasentransferkatalysators bei Temperaturen von 40 bis 170°C, bevorzugt bei 60 bis 130°C, hergestellt werden.

Bevorzugte Verbindungen der Formel (IX) sind solche, in denen R⁴ für C(CH₃)₂-OH oder Si(CH₃)₃ steht.

Bevorzugte Verbindungen der Formel (V) sind solche, wie sie bei der Formel (II) genannt wurden.

Für den Palladium-Katalysator, den Co-Katalysator, die Base, das Verdünnungs- und/oder Lösungsmittel, den Zusatz von Phosphan und Phasentransferkatalysator gilt das gleiche wie bei der zuvor genannten Variante beschriebene.

Auf 1 Mol des Acetylens der Formel (IX) setzt man im allgemeinen 0,8 bis 1,2 Mol, vorzugsweise 0,9 bis 1,1 Mol, des Arylhalogenids der Formel (V) ein. Die Umsetzung der Verbindungen der Formel (IX) mit denen der Formel (V) kann in der gleichen Weise geschehen wie bei der zuvor beschriebenen Variante ausgeführt.

Formelmäßig wird diese Variante beispielhaft anhand der Synthese von Z,E-1,4-Bis-(4-carboxystyryl)benzol dargestellt: 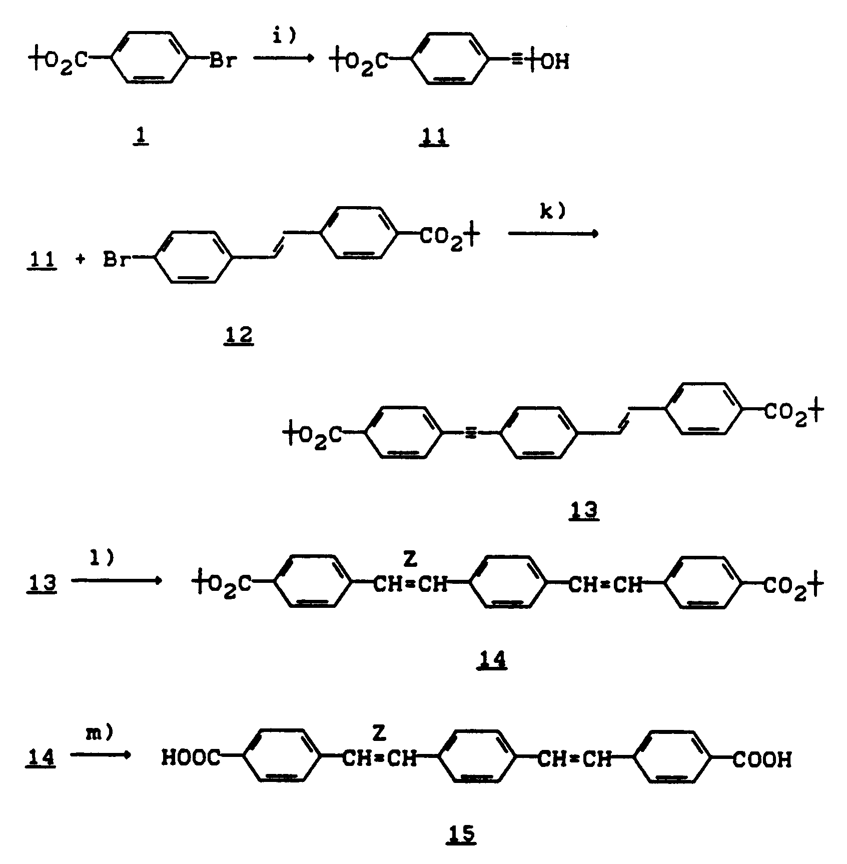
i) ≡┼OH, (Pφ₃)₂PdCl₂, CuI, NEt₃, Pφ₃, CH₃CN
k) (φ₃P)₂PdCl₂, CuI, Pφ₃, NaOH, H₂O, Toluol, BzNEt₃Cl
l) Zn/Cu EtOH/THF
m) 1. p-TsOH, Toluol 2. NaOH, H₂O, EtOH 3. HCl

Die Umsetzungen m) 2. und 3. dienen der Reinigung des Reaktionsproduktes.

Ein weiterer Gegenstand der vorliegenden Anmeldung sind neue Z-Stilbenverbindungen der Formeln 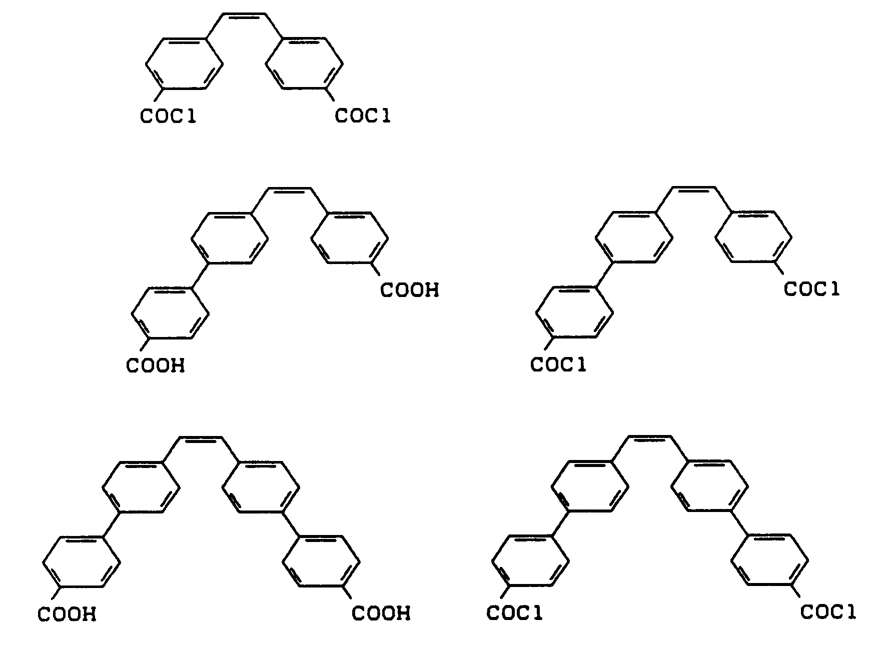 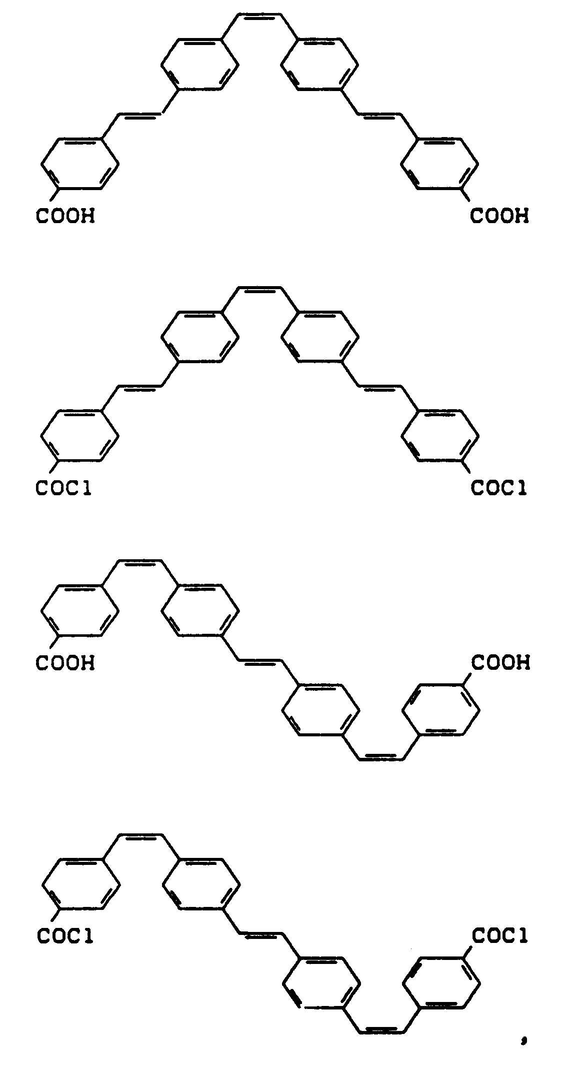
bevorzugt

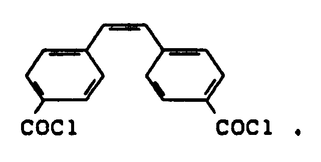

Diese neuen Z-Stilbenverbindungen können nach dem zuvor beschriebenen erfindungsgemäßen Verfahren oder dessen Varianten hergestellt werden.

Die nach dem erfindungsgemäßen Verfahren hergestellten bifunktionellen Z-Stilbenverbindungen eignen sich in sehr guter Weise zur Herstellung von Polymeren, wie Polyester, Polyestercarbonaten, Polyamiden, Polycarbonaten, Polyurethanen, Polyethern und Polyphenylensulfiden. Durch ihre besonderen Eigenschaften zeichnen sie sich vorteilhaft aus gegenüber bekannten isomeren Stilbenderivaten, die bislang zum Aufbau Stilben-Fragmente enthaltender Polymerer verwendet wurden (siehe z.B. US 4 654 412). So sind die Löslichkeitsunterschiede von bifunktionellen Z- und E-Stilben-Monomeren in Lösungsmitteln, die zur Herstellung von Polymeren durch Phasengrenzflächenkondensation eingesetzt werden, beträchtlich.

So ist es z.B. möglich, eine 11-%ige Lösung von Z-Stilben-4,4′-dicarbonsäuredichlorid in Dichlormethan herzustellen, während die Löslichkeit des Chlorids der bekannten E-Stilben-4,4′-dicarbonsäure weniger als 1 % beträgt und deshalb - im Gegensatz zur gut löslichen Z-Stilbenverbindung - nicht in einer wirtschaftlich vertretbaren Phasengrenzflächenkondensation eingesetzt werden kann. Offenbar sind aus diesem Grunde Verfahren zur Herstellung von Polystilbendicarbonsäureestern nach dem Phasengrenzflächenkondensationsverfahren bislang nicht bekannt geworden. Mit den erfindungsgemäß hergestellten, zum Teil neuen bifunktionellen Z-Stilbenverbindungen wurde demnach eine wesentliche Bereicherung der Herstellungstechnik von bekannten und neuen Polystilbenfragmente enthaltenden Werkstoffen erreicht, die sich z.B. durch hohe Temperaturbeständigkeit auszeichnen.

### Beispiele

### Beispiel 1

### 4-(Trimethylsilylethinyl)-benzoesäure-tert.-butylester

Unter N₂ rührt man 51,4 g Brombenzoesäure-tert.-butylester, 21,6 g Trimethylsilylacetylen, 1,4 g (Ph₃P)₂PdCl₂ in 400 ml abs. Acetonitril und 100 ml Triethylamin 8 h unter Rückfluß. Nach Aufarbeitung mit CH₂Cl₂/H₂O und Destillation erhält man 40,6 g 4-(Trimethylsilylethinyl)-benzoesäure-tert-butylester.
Kp._{0,04}: 120-130°C
Fp.: 60-62°C
NMR (CDCl₃): δ = 0,28 (s, 9H), 1,63 (s, 9H), 7,53 (m, 2H), 7,95 (m, 2H)

### Beispiel 2

### 4-Ethinyl-benzoesäure-tert.-butylester

Unter N₂ rührt man 274,4 g 4-(Trimethylsilylethinyl)benzoesäure-tert.-butylester und 12,5 g wasserfreies Kaliumcarbonat in 1,5 l abs Methanol (3 h). Man dampft ein, arbeitet mit CH₂Cl₂/H₂O auf und erhält 194 g 4-Ethinyl-benzoesäure-tert.-butylester.
Fp.: 70-71°C
NMR (CDCl₃): δ = 1,58 (s, 9H), 3,22 (s, 1H), 7,52 (m, 2H) 7,94 (m, 2H)

### Beispiel 3

### 4,4′-Tolandicarbonsäuredi-tert.-butylester

Unter N₂ legt man 193,5 g 4-Brom-benzoesäure-tert.-butylester, 5,1 g Triphenylphosphin und 375 ml NEt₃ in 1 l abs. Acetonitril vor, leitet 20 min N₂ durch die Lösung, gibt dann 10,5 g (Pφ₃)₂PdCl₂ und 1,5 g Kupfer(I)iodid zu und tropft bei Rückfluß langsam 166,5 g 4-Ethinyl-benzoesäure-tert.-butylester gelöst in 1 l abs. Acetonitril zu und rührt über Nacht. Nach dem Abkühlen wird abgesaugt, in H₂O verrührt, wieder abgesaugt, in CH₂Cl₂ gelöst und nach Zugabe von etwas Tonsil filtriert und das Lösungsmittel im Vakuum entfernt. Man erhält 225 g 4,4′-Tolandicarbonsäuredi-tert.-butylester.
Fp.: 163°C
NMR (CDCl₃): δ = 1,60 (s, 18H), 7,58 (m, 4H), 7,98 (m, 4H)

### Beispiel 4

### 4,4′-Tolandicarbonsäuredi-tert.-butylester

Eine Mischung von 514 g 4-Brombenzoesäure-tert.-butylester, 88,25 g 2-Methyl-3-butin-2-ol und 74,5 g Triphenylphosphin in 1250 ml Toluol gibt man zu 7,1 g Benzyltriethylammoniumchlorid, 9,55 g Kupfer(I)iodid und 14 g (Pφ₃)₂PdCl₂ und rührt nach Zugabe von 750 ml 5,5 N NaOH 48 h bei Rückfluß. Die organische Phase wird abgetrennt, getrocknet und eingedampft. 430 g feuchtes Rohprodukt werden in 550 ml Methanol verrührt und abgesaugt. Man erhält 248 g 4,4′-Tolandicarbonsäuredi-tert.-butylester, der sich durch Umkristallisation aus Acetonitril weiter reinigen läßt.

Fp. und NMR-Daten stimmen mit denen des nach Beispiel 3 erhaltenen Produkts überein.

### Beispiel 5

### Z-4,4′-Stilbendicarbonsäuredi-tert.-butylester

52,6 g Zn und 70 ml Ethanol werden mit 13 g 1,2-Dibromethan erhitzt, bis die Gasentwicklung beendet ist. Bei 40 bis 50°C gibt man eine Lösung von 13 g CuBr und 15,8 g wasserfreiem LiBr in 70 ml THF zu und rührt 10 min. Dazu gibt man eine heiße Lösung von 50 g 4,4′-Tolandicarbonsäuredi-tert.-butylester in 70 ml Ethanol und 130 ml THF und erhitzt 72 h auf Rückfluß. Nach dem Filtrieren wird eingeengt, in CH₂Cl₂ aufgenommen, je 2 x mit verd. HCl und H₂O gewaschen, getrocknet und eingedampft. Man erhält 46,3 g Z-4,4′-Stilbendicarbonsäureditert.-butylester.
Fp.: 126-127°C
NMR (CDCl₃): δ = 1,58 (s, 18H), 6,69 (s, 2H), 7,24 (m, 4H), 7,84 (m, 4H)

### Beispiel 6

### Z-4,4′-Stilbendicarbonsäure

331 g Z-4,4′-Stilbendicarbonsäuredi-tert.-butylester werden in 1700 ml abs. Toluol mit 10,3 g p-Toluolsulfonsäure 3 h am Rückfluß gekocht. Es wird eingedampft, mit 104,2 g NaOH, 1300 ml H₂O und 1300 ml Ethanol 4 h am Rückfluß gekocht. Man filtriert heiß nach Zugabe von Aktivkohle, verdünnt mit ca. 3 l H₂O, extrahiert 2 x mit je 1 l CH₂Cl₂ und versetzt die wäßrige Phase mit konz. HCl. Nach Absaugen und Trocknen erhält man 230 g Z-4,4′-Stilbendicarbonsäure.
Fp.: >280°C
NMR (DMSO-d₆): δ = 6,83 (s, 2H), 7,35 (m, 4H), 7,86 (m, 4H)

### Beispiel 7

### Z-4,4′-Stilbendicarbonsäuredichlorid

100 g Z-Stilben-4,4′-dicarbonsäure werden mit 400 ml Thionylchlorid und 3 Tropfen DMF bei 40°C bis zum Ende der Gasentwicklung gerührt. Man filtriert unter Stickstoff und dampft zur Trockne ein. Man erhält 95 g Z-4,4′-Stilbendicarbonsäuredichlorid.
Fp.: 102°C
NMR (CDCl₃): δ = 6,81 (s, 2H), 7,34 (m, 4H), 8,00 (m, 4H)
Ein noch saubereres Produkt erhält man, indem man anschließend aus einem inerten Lösungsmittel, wie z.B. Cyclohexan, Benzol oder Toluol, umkristallisiert und das erhaltene Produkt gegebenenfalls nochmal mit Thionylchlorid, gegebenenfalls in einem geeigneten Lösungsmittel, wie CH₂Cl₂, behandelt.

### Beispiel 8

### 4-(4-Carbo-tert.-butoxyphenyl)-2-methyl-3-butin-2-ol

Unter N₂ rührt man 257,1 g 4-Brombenzoesäure-tert.-butylester, 168 g 2-Methyl-3-butin-2-ol und 15,6 g (Ph₃P)₂PdCl₂ in 1,5 l abs. Acetonitril und 480 ml Triethylamin 6 h unter Rückfluß. Nach dem Eindampfen wird mit Toluol/H₂O aufgearbeitet, man erhält 185 g eines Öls, das langsam kristallisiert.
NMR (CDCl₃): δ = 1,60 (s, 9H), 1,65 (s, 6H), 7,43 (m, 2H), 7,92 (m, 2H)

### Beispiel 9

### E-4-(Carbo-tert.-butoxy)-4′-(4-carbo-tert.-butoxystyryl)-tolan

Eine Mischung von 13,02 g 4-(4-Carbo-tert.-butoxyphenyl)-2-methyl-3-butin-2-ol, 17,97 g 4-Brom-4′-carbotert.-butoxystilben, 0,93 g Triphenylphosphin, 0,34 g Benzyltriethylammoniumchlorid, 175 mg Bis(triphenylphosphin)palladium(II)chlorid, 175 mg Kupfer(I)iodid in 70 ml Toluol und 36 ml 5,5 N NaOH wird 48 h kräftig unter Rückfluß gerührt. Nach Zugabe von 250 ml gesättigter Ammoniumchloridlösung wird mit Methylenchlorid extrahiert, getrocknet und eingedampft.
Ausbeute: 22,1 g
Charakterisierung s. Beispiel 10.

### Beispiel 10

### E-4-(Carbo-tert.-butoxy)-4′-(4-carbo-tert.-butoxystyryl)-tolan

Unter N₂ gibt man zu 234 g 4-Brom-4′-carbo-tert.-butoxystilben in 1 l abs. Acetonitril 300 ml NEt₃ und 20,46 g Pφ₃. Man leitet 20 min N₂ durch die gerührte Suspension und gibt dann 9,12 g (Pφ₃)₂PdCl₂ und 2,5 g Kupfer(I)iodid zu. Bei Rückfluß tropft man dann über einen Zeitraum von 6 h 144 g 4-Ethinylbenzoesäure-tert.-butylester gelöst in 1 l abs. Acetonitril zu und kocht über Nacht unter Rückfluß. Das heiß abgesaugte Produkt wird in CH₂Cl₂ gelöst, man wäscht mehrmals mit H₂O, trocknet und entfernt das Lösungsmittel.
Ausbeute: 275 g
Fp.: 218°C (Zers.)
NMR (CDCl₃): δ = 1,61 (s, 18H), 7,18 (s, 2H), 7,56 (m, 8H), 7,98 (m, 4H)

### Beispiel 11

### Z,E-1,4-Bis-(4-carbo-tert.-butoxystyryl)-benzol

272 g Zn-Staub in 270 ml tert.-Butanol werden mit 24,9 ml 1,2-Dibromethan bis zum Ende der Gasentwicklung gekocht. Dann gibt man vorsichtig bei 50°C 55,5 g CuBr und 66,9 g LiBr in 200 ml THF zu. Wenn die exotherme Reaktion abgeklungen ist, versetzt man mit 272 g E-4-(Carbo-tert.-butoxy)-4′-(4-carbo-tert.-butoxy-styryl)tolan in 1,7 l THF und 560 ml tert.-Butanol und erhitzt 4 Tage unter Rückfluß. Dann wird zur Trockne eingedampft, in CH₂Cl₂ aufgenommen, filtriert, das Filtrat mit verd. HCl gewaschen, getrocknet und eingedampft.
Ausbeute: 247 g
Fp.: 125°C
NMR (CDCl₃): δ = 1,59 (s, 9H), 1,61 (s, 9H), 6,65 (m, 2H), 7,13 (m, 2H), 7,23 (m, 2H), 7,31 (m, 2H), 7,40 (m, 2H), 7,51 (m, 2H), 7,86 (m, 2H), 7,96 (m, 2H)

### Beispiel 12

### Z,E-1,4-Bis-(4-carboxy-styryl)-benzol

131 g Z,E-1,4-Bis-(4-carbo-tert.-butoxy-styryl)-benzol werden 3 h mit 6,5 g p-Toluolsulfonsäure in 1 l Toluol unter Rückfluß gekocht. Man engt zur Trockne ein und erhitzt weiter 4 h mit 30 g NaOH in 300 ml Wasser und 600 ml Ethanol. Das Ethanol wird abdestilliert und der Rückstand nach Verdünnen mit Wasser angesäuert (konz. HCl). Man saugt ab und trocknet.
Ausbeute: 96 g
NMR (DMSO-d₆): δ = 6,74 (m, 2H), 7,25 (m, 2H), 7,30-7,45 (m, 4H), 7,54 (m, 2H), 7,70 (m, 2H), 7,86 (m, 2H), 7,96 (m, 2H), 12,9 (breit, OH)

### Beispiel 13

### 1-(4-Carbo-tert.-butoxyphenyl)-2-(4′-carboethoxybiphenyl-4-yl)-ethin

Unter N₂ gibt man zu 61 g 4-Brom-4′-carboethoxybiphenyl in 300 ml abs. Acetonitril, 100 ml Triethylamin, 6,3 g Triphenylphosphin, 2,81 g (Pφ₃)₂PdCl₂ und 0,77 g Kupfer(I)iodid. Bei Rückflußtemperatur tropft man langsam 44,3 g 4-Ethinylbenzoesäure-tert.-butylester gelöst in 300 ml abs. Acetonitril zu und kocht über Nacht. Man saugt heiß ab, verrührt in H₂O, saugt ab, löst den Rückstand in CH₂Cl₂ und filtriert nach Zugabe von Tonsil.
Ausbeute: 73,2 g
Fp.: 169-170°C
NMR (CDCl₃): δ = 1,41 (t, 3H), 1,60 (s, 9H), 4,41 (q, 2H), 7,58 (m, 2H), 7,63 (s, 4H), 7,68 (m, 2H), 7,98 (m, 2H), 8,13 (m, 2H)

### Beispiel 14

### Z-1-(4-Carbo-tert.-butoxyphenyl)-2-(4′-carboethoxybiphenyl-4-yl)-ethen

50,6 g Zink, 54 ml abs. Ethanol und 10,7 ml 1,2-Dibromethan werden bis zum Ende der Gasentwicklung gekocht. Bei 50°C gibt man dann eine Lösung von 10,7 g CuBr und 12,7 g LiBr in 54 ml abs. THF und nach 5 min 47,8 g 1-(4-Carbo-tert.-butoxyphenyl)-2-(4′-carboethoxybiphenyl-4-yl)-ethin in 100 ml abs. THF und 30 ml abs. Ethanol zu und kocht 5 Tage unter Rückfluß. Man saugt ab, engt ein und arbeitet mit CH₂Cl₂/1 N HCl auf.
Ausbeute: 31,2 g
NMR (CDCl₃): δ = 1,40 (t, 3H), 4,40 (q, 2H), 6,72 (m, 2H), 7,31 (m, 2H), 7,38 (m, 2H), 7,53 (m, 2H), 7,65 (m, 2H), 8,00 (m, 2H), 8,10 (m, 2H).
Signale überlagern sich zum Teil.

### Beispiel 15

### Z-1-(4-Carboxyphenyl)-2-(4′-carboxybiphenyl-4-yl)-ethen

31,2 g 1-(4-Carbo-tert.-butoxyphenyl)-2-(4′-carboethoxybiphenyl-4-yl)-ethen werden in 150 ml abs. Toluol mit 1 g p-Toluolsulfonsäure 3 h am Rückfluß gekocht. Man engt vollständig ein, versetzt mit 8,68 g NaOH in 100 ml H₂O und 100 ml Ethanol und kocht 4 h. Man filtriert heiß nach Zugabe von Aktivkohle, verdünnt mit 200 ml H₂O, extrahiert 2 x mit CH₂Cl₂ und fällt das Produkt aus der wäßrigen Phase mit konz. HCl aus.
Ausbeute: 17,75 g
NMR (DMSO-d₆): δ = 6,78 (m, 2H), 7,34 (m, 2H), 7,40 (m, 2H), 7,67 (m, 2H), 7,80 (m, 2H), 7,88 (m, 2H), 8,04 (m, 2H), 12,91 (breit, OH)

### Beispiel 16

### 4-Bromtolan-4′-carbonsäure-tert.-butylester

Zur Lösung von 40,4 g 4-Ethinylbenzoesäure-tert.-butylester, 56,6 g Bromiodbenzol und 1,34 g Ph₃P in 50 ml Diethylamin und 300 ml abs. Acetonitril setzt man 2,8 g (Ph₃P)₂PdCl₂ und 400 mg CuI zu, rührt 72 h bei 40°C, saugt ab und trocknet.
Ausbeute: 50,5 g
Fp.: 115 - 116°C
NMR (CDCl₃): δ = 1,59 (s, 9H), 7,3-7,6 (m, 6H), 7,96 (m, 2H)

### Beispiel 17

### Z-4-Brom-4′-carbo-tert.-butoxystilben

56 g Zn und 14 g 1,2-Dibromethan werden in 70 ml Ethanol bis zum Ende der Gasentwicklung gekocht, anschließend gibt man zunächst 14 g Kupferbromid und 16,8 g Lithiumbromid in 70 ml THF und dann 50 g 4-Bromtolan-4′-carbonsäure-tert.-butylester in 250 ml THF und 50 ml Ethanol zu und kocht 44 h unter Rückfluß. Man filtriert, engt ein, nimmt in CH₂Cl₂ auf, wäscht mit verdünnter Salzsäure, trocknet und dampft ein.
Ausbeute: 40 g, etwas verunreinigt mit Z-4-Stilbencarbonsäure-tert.-butylester.

### Beispiel 18

### Z,E,Z-1,2-Bis-(4-carbo-tert.-butoxystilbenyl-4′-)-ethen

Bei 100°C leitet man 8h lang Ethylen durch die Lösung von 17,9 g Z-4-Brom-4′-carbo-tert.-butoxystilben, 9,82 g Kaliumacetat, 0,91 g Triphenylphosphin und 0,11 g (Pφ₃)₂PdCl₂ in 150 ml abs. DMF. Man verdünnt mit Wasser, extrahiert mit Ether, wäscht mehrmals gut mit Wasser, trocknet, engt ein und verrührt den Rückstand in Petrolether.
Ausbeute: 3,2 g
NMR (CDCl₃): δ = 1,55 (s, 18H), 7,62 (m, 4H), 7,02 (s, 2H), 7,15-7,45 (m, 12H), 7,86 (m, 4H)

## Patentansprüche

1. Verfahren zur Herstellung von bifunktionellen, mindestens eine Z-konfigurierte Stilbengruppierung enthaltenden Stilbenverbindungen der Formel 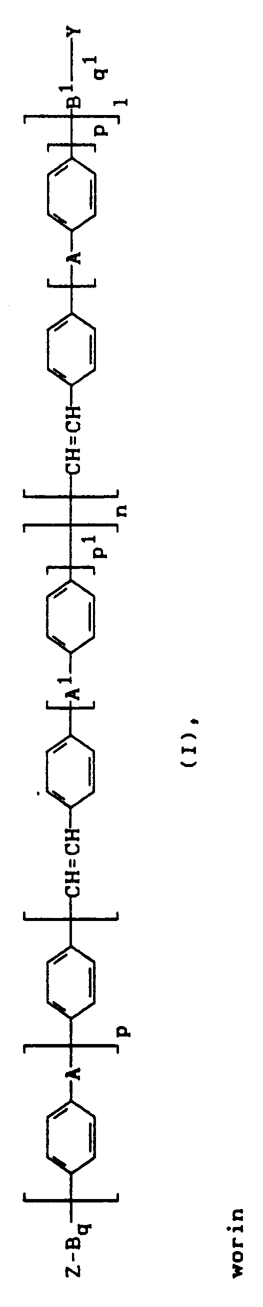
Z und Y gleich oder verschieden sind und für COOR, COR, COCl, OCOCl, NCO, NRCOCl, NHR, OR oder Cl stehen mit R = Wasserstoff oder C₁-C₈-Alkyl,
A und A¹ gleich oder verschieden sind und für eine chemische Bindung oder CH=CH stehen,
B 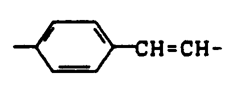 und
B¹ 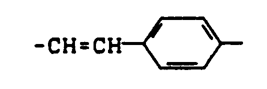 bedeuten,
l 0 oder 1,
p und p¹ 0 oder 1,
q und q¹ 0 oder 1 und
n 0, 1 oder 2 bedeuten,
dadurch gekennzeichnet, daß man Verbindungen der Formel 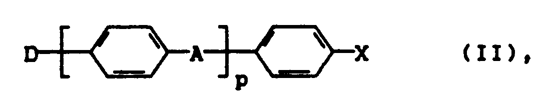 worin
A und p die obengenannte Bedeutung besitzen,
D COOR¹, COR¹, NHR², NO₂, OR³, Cl oder Br bedeutet mit R¹ = Wasserstoff oder verzweigtes oder geradkettiges C₁-C₈-Alkyl, R² = OAc, COOC₂H₅ oder eine andere geeignete Schutzgruppe und R³ = OAc, 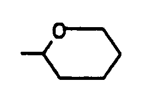 oder eine andere geeignete Schutzgruppe,
und
X für Brom oder Iod steht,
mit einem Acetylen der Formel
HC≡C-R⁴ (III),
worin
R⁴ für 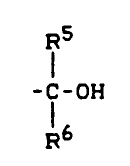 oder -Si(R⁵)₃ steht, mit R⁵ und R⁶ = C₁-C₄-Alkyl,
in Gegenwart eines Palladium-Katalysators und einer Base bei Temperaturen von 30 bis 160°C umsetzt, die erhaltenen Verbindungen in Gegenwart einer Base in Verbindungen der Formel 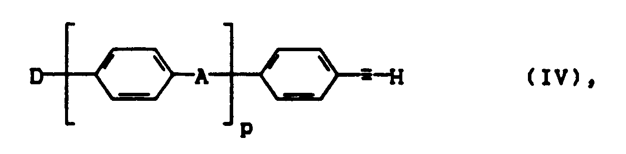 worin
D, A und p die obengenannte Bedeutung haben,
überführt, danach die Verbindungen der Formel (IV) mit Verbindungen der Formel 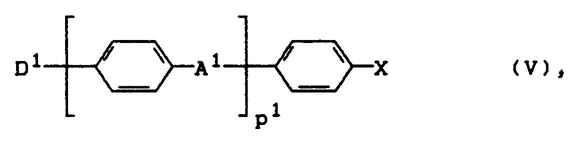 worin
D¹, A¹ und p¹ die für D, A und p beschriebene Bedeutung besitzen und
X für Brom oder Iod steht,
in Gegenwart eines Palladium-Katalysators und einer Base bei Temperaturen von 30 bis 160°C umsetzt, anschließend die erhaltenen Acetylen-Verbindungen in Gegenwart von Katalysatoren mit Wasserstoff oder mit anderen Reduktionsmitteln zu Z-Stilbenverbindungen der Formel 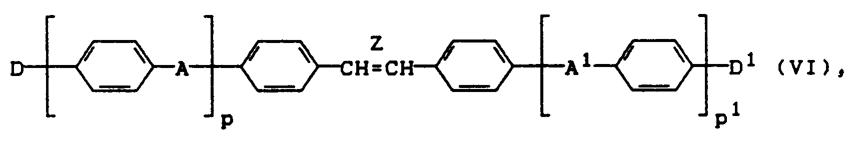 worin
D, D¹, A, A¹ und p, p¹ die obengenannte Bedeutung haben,
reduziert und die funktionellen Gruppen D und D¹ in die obengenannten funktionellen Gruppen Z und Y der Formel (I) in bekannter Weise überführt
oder
die Z-Stilbenverbindungen der Formel (VI) im Fall daß D und D¹ mindestens für ein Bromatom stehen mit Verbindungen der Formel
H₂C=CHR⁷ (VII),
worin
R⁷ für Wasserstoff oder 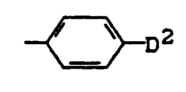 steht, wobei
D² die für D¹ genannte Bedeutung besitzt,
in Gegenwart eines Palladium-Katalysators und einer Base bei Temperaturen von 30 bis 160°C umsetzt und die funktionellen Gruppen D, D¹ und D² in die funktionellen Gruppen Z und Y der Formel (I) in üblicher Weise überführt
oder
die Verbindungen der Formel (IV) mit Verbindungen der Formel 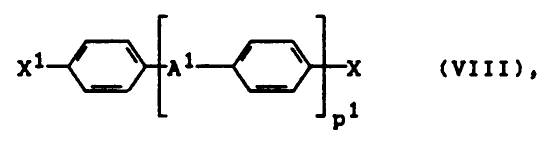 worin
A¹, X und p¹ die zuvor genannte Bedeutung besitzen und
X¹ für Brom oder Iod steht,
in Gegenwart eines Palladium-Katalysators und einer Base bei Temperaturen von 30 bis 160°C umsetzt, anschließend die erhaltenen Acetylenverbindungen in Gegenwart von Katalysatoren mit Wasserstoff oder mit anderen Reduktionsmitteln zu entsprechenden Z-Stilbenverbindungen reduziert und danach die funktionellen Gruppen D in die funktionellen Gruppen Z und Y der Formel (I) in bekannter Weise überführt.

2. Neue Z-Stilbenverbindungen der Formeln

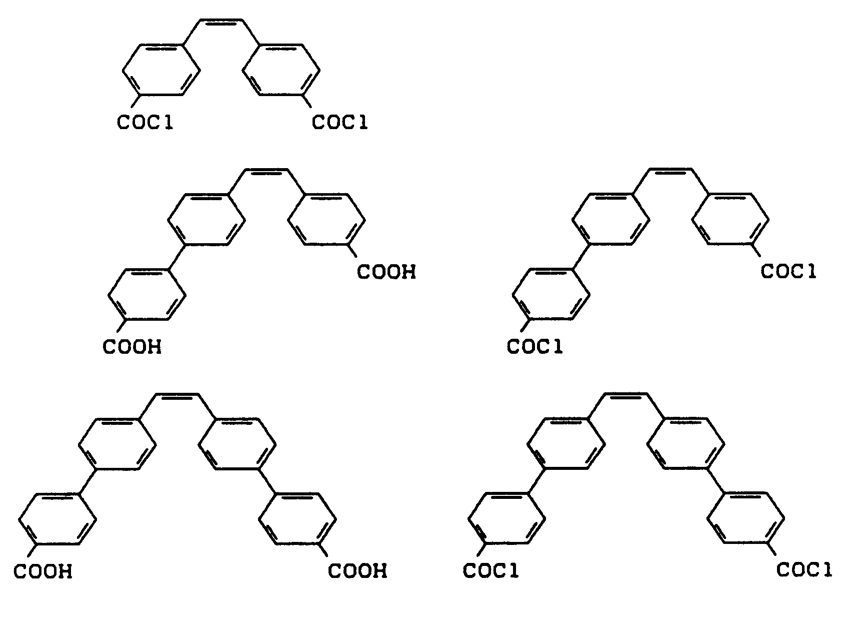

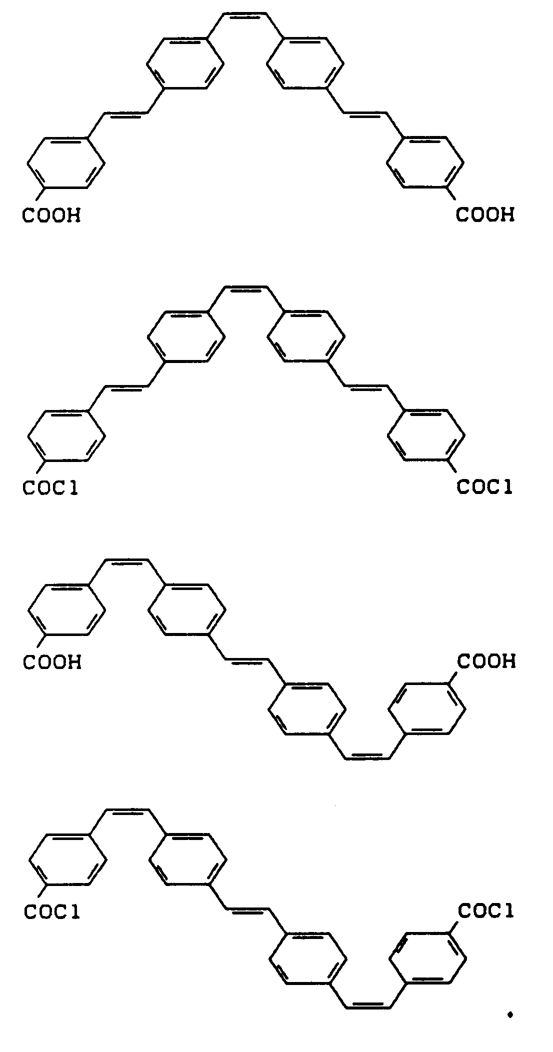

3. Verwendung der nach Anspruch 1 hergestellten bifunktionellen Z-Stilbenverbindungen zur Herstellung von Polymeren.

## Claims

1. A process for the production of bifunctional stilbene compounds containing at least one Z-configured stilbene group and corresponding to the following formula: 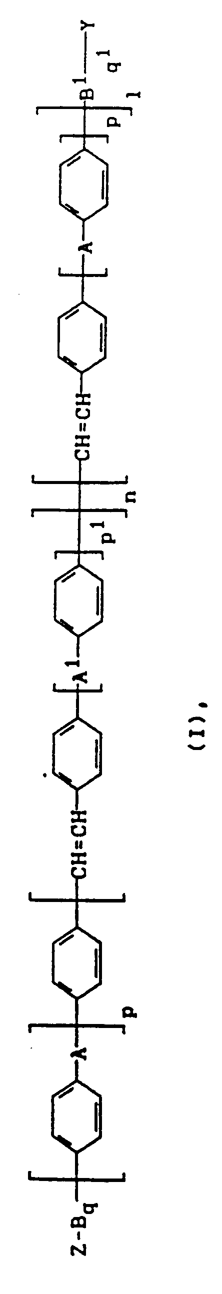 in which
Z and Y may be the same or different and represent COOR, COR, COCl, OCOCl, NCO, NRCOCl, NHR, OR or Cl where R = hydrogen or C₁₋₈ alkyl,
A and A¹ may be the same or different and represent a chemical bond or CH=CH,
B represents 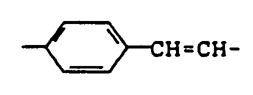 and
B¹ represents 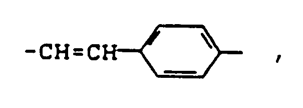
l = 0 or 1,
p and p¹ = 0 or 1,
q and q¹ = 0 or 1 and
n = 0, 1 or 2,
characterized in that compounds corresponding to the following formula: 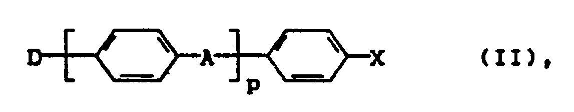 in which
A and p are as defined above,
D represents COOR¹, COR¹, NHR², NO₂, OR³, Cl or Br where R¹ = hydrogen or linear or branched C₁₋₈ alkyl, R² = OAc, COOC₂H₅ or another suitable protective group and R³ = OAc, 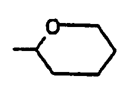 or another suitable protective group
and
X represents bromine or iodine,
are reacted with an acetylene corresponding to the following formula:
HC≡C-R⁴ (III)
in which
R⁴ represents 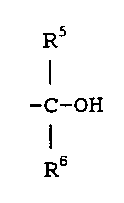 or -Si(R⁵)₃ where R⁵ and R⁶ = C₁₋₄ alkyl,
in the presence of a palladium catalyst and a base at temperatures in the range from 30 to 160°C, the compounds obtained are converted in the presence of a base into compounds corresponding to the following formula: 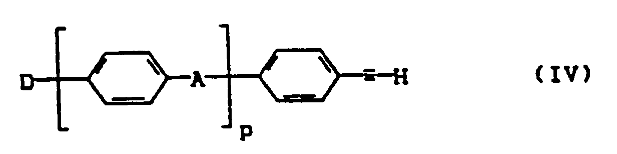 in which
D, A and p are as defined above,
after which the compounds corresponding to formula (IV) are reacted with compounds corresponding to the following formula: 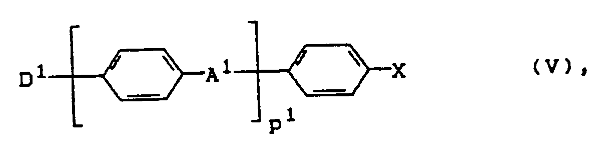 in which
D¹, A¹ and p¹ have the meanings defined for D, A and p and
X represents bromine or iodine,
in the presence of a palladium catalyst and a base at temperatures in the range from 30 to 160°C and the acetylene compounds obtained are reduced with hydrogen or with other reducing agents in the presence of catalysts to form Z-stilbene compounds corresponding to the following formula: 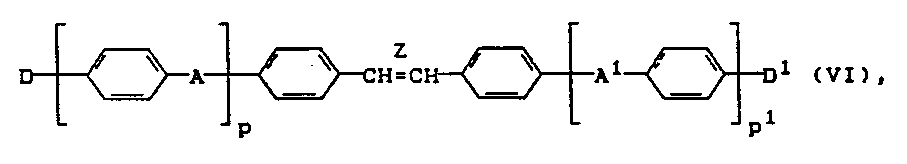 in which
D, D¹, A, A¹ and p, p¹ are as defined above,
and the functional groups D and D¹ are converted in known manner into the above-mentioned functional groups Z and Y corresponding to formula (I)
or
where D and D¹ represent at least one bromine atom, the Z-stilbene compounds corresponding to formula (VI) are reacted with compounds corresponding to the following formula:
H₂C=CHR⁷ (VII)
in which
R⁷ represents hydrogen or 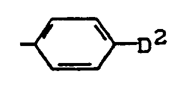 where D² has the meaning defined for D¹,
in the presence of a palladium catalyst and a base at temperatures in the range from 30 to 160°C and the functional groups D, D¹ and D² are converted by standard methods into the functional groups Z and Y in formula (I) or
the compounds corresponding to formula (IV) are reacted with compounds corresponding to the following formula: 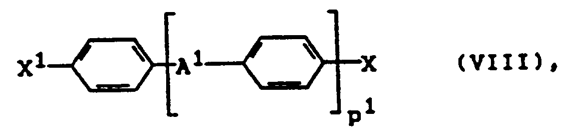 in which
A¹, X and p¹ are as defined above and
X¹ represents bromine or iodine,
in the presence of a palladium catalyst and a base at temperatures in the range from 30 to 160°C, the acetylene compounds obtained are reduced with hydrogen or with other reducing agents in the presence of catalysts to form corresponding Z-stilbene compounds and the functional groups D are subsequently converted in known manner into the functional groups Z and Y in formula (I).

2. New Z-stilbene compounds corresponding to the following formulae:

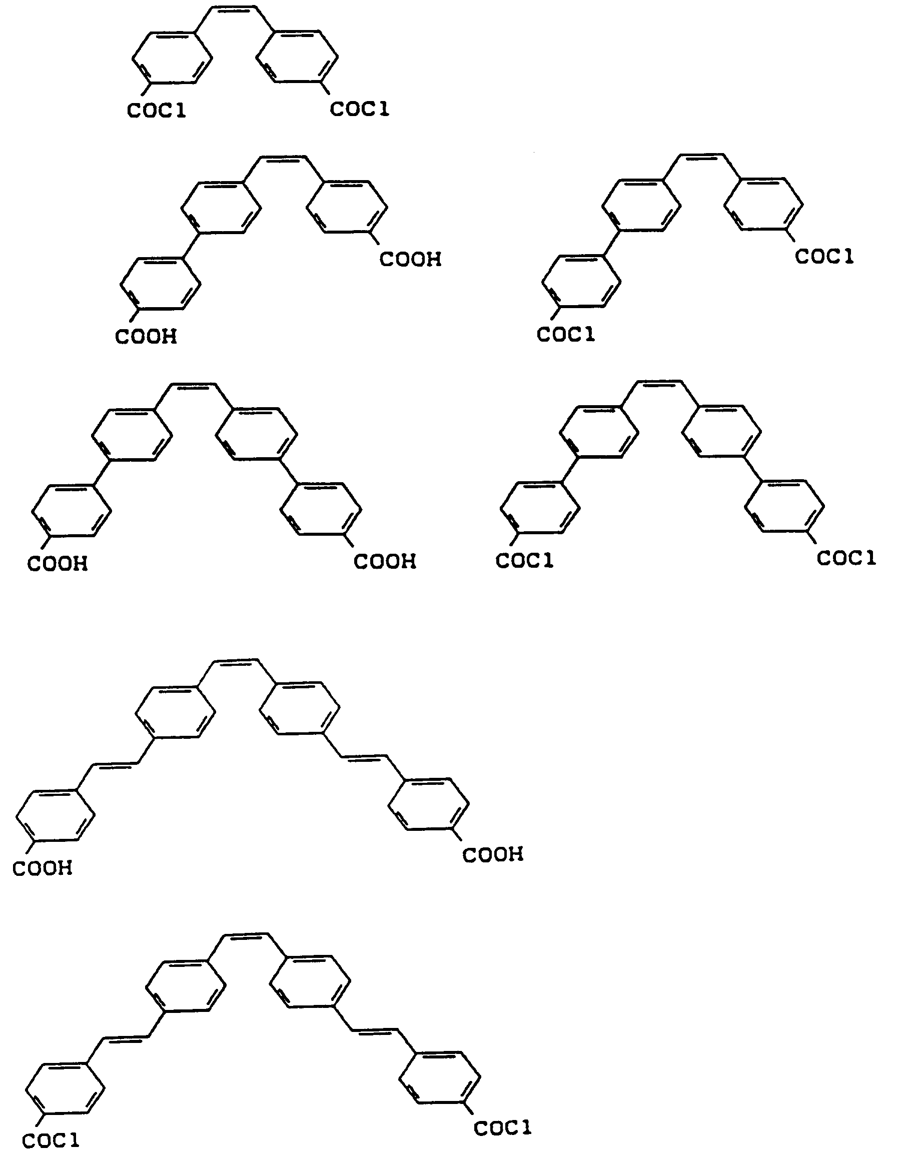

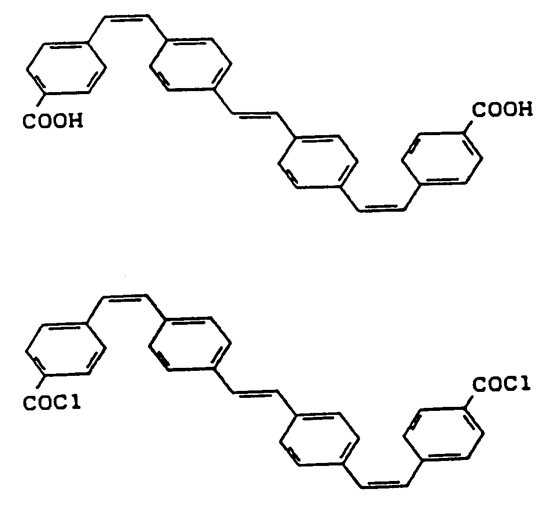

3. The use of the bifunctional Z-stilbene compounds produced by the process claimed in claim 1 for the production of polymers.

## Revendications

1. Procédé de production de composés de stilbène bifonctionnels contenant au moins un groupement stilbène à configuration Z, de formule 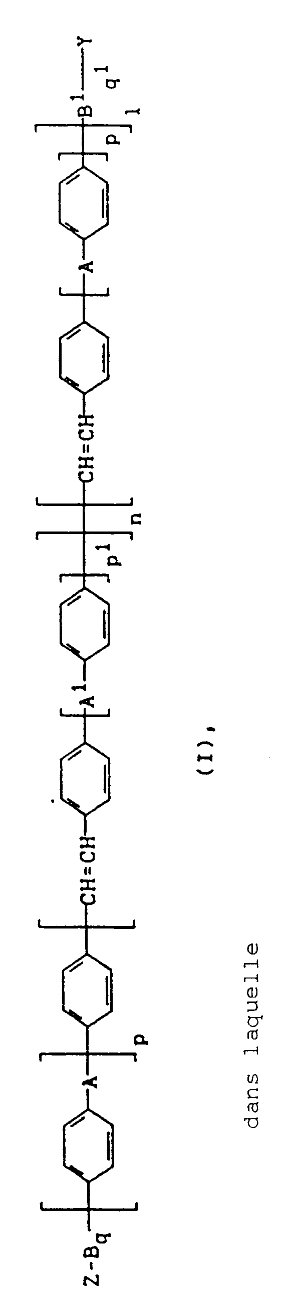 où
Z et Y sont identiques ou différents et représentent un groupe COOR, COR, COCl, OCOCl, NCO, NRCOCl, NHR, OR ou Cl, avec R = hydrogène ou groupe alkyle en C₁ à C₈,
A et A¹ sont identiques ou différents et représentent une liaison chimique ou un groupe CH=CH,
B représente 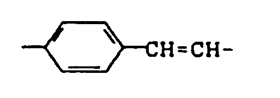 et
B¹ représente 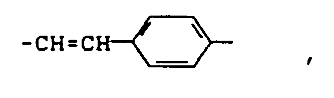
l a la valeur 0 ou 1,
p et p¹ ont la valeur 0 ou 1,
q et q¹ ont la valeur 0 ou 1 et
n a la valeur 0, 1 ou 2,
caractérisé en ce qu'on fait réagir des composés de formule 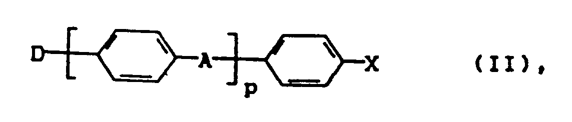 dans laquelle
A et p ont la définition indiquée ci-dessus,
D représente COOR¹, COR¹, NHR², NO₂, OR³, Cl ou Br avec R¹ = hydrogène ou groupe alkyle en C₁ à C₈ ramifié ou linéaire, R² = OAc, COOC₂H₅ ou un autre groupe protecteur approprié et R³ = OAc, 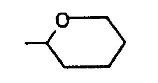 ou un autre groupe protecteur approprié,
et
X représente du brome ou de l'iode,
avec un composé acétylénique de formule
HC≡C-R⁴ (III),
dans laquelle
R⁴ représente 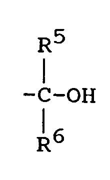 ou -Si(R⁵)₃_{,} avec R⁵ et R⁶ = alkyle en C₁ à C₄,
en présence d'un catalyseur au palladium et d'une base à des températures de 30 à 160°C, on transforme les composés obtenus en présence d'une base en composés de formule 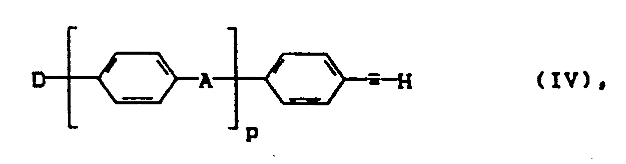 dans laquelle
D, A et p ont la définition indiquée ci-dessus,
puis on fait réagir les composés de formule (IV) avec des composés de formule 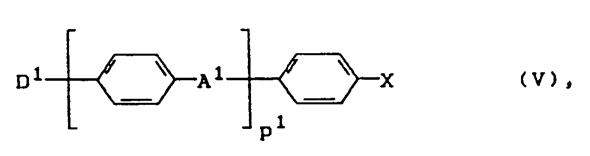 dans laquelle
D¹, A¹ et p¹ ont la définition indiquée pour D, A et p et
X représente du brome ou de l'iode,
en présence d'un catalyseur au palladium et d'une base à des températures de 30 à 160°C, puis on réduit les composés acétyléniques obtenus en présence de catalyseurs avec de l'hydrogène ou un autre agent réducteur en composés de Z-stilbène de formule 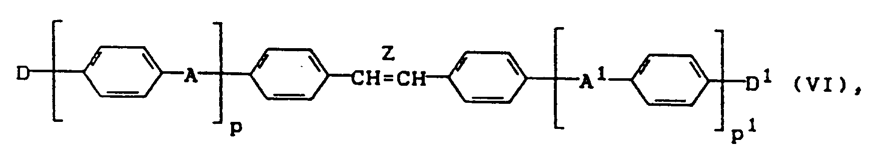 dans laquelle
D, D¹, A, A¹ et p, p¹ ont la définition indiquée ci-dessus,
et on transforme les groupes fonctionnels D et D¹ en les groupes fonctionnels Z et Y mentionnés ci-dessus de formule (I) d'une manière connue,
ou bien
on fait réagir les composés de Z-stilbène de formule (VI) au cas où D et D¹ représentent au moins un atome de brome, avec des composés de formule
H₂C=CHR⁷ (VII),
dans laquelle
R⁷ représente de l'hydrogène ou un groupe 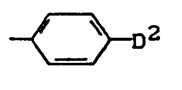 où
D² a la définition mentionnée pour D¹,
en présence d'un catalyseur au palladium et d'une base à des températures de 30 à 160°C et on transforme les groupes fonctionnels D, D¹ et D² d'une manière classique en les groupes fonctionnels Z et Y de formule (I)
ou bien
on fait réagir les composés de formule (IV) avec des composés de formule 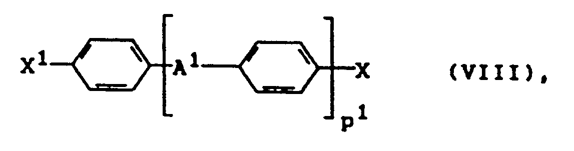 dans laquelle
A¹, X et p¹ ont la définition indiquée précédemment et
X¹ représente du brome ou de l'iode,
en présence d'un catalyseur au palladium et d'une base à des températures de 30 à 160°C, puis on réduit les composés acétyléniques obtenus en présence de catalyseurs avec de l'hydrogène ou avec d'autres agents réducteurs en composés de Z-stilbène correspondants et on transforme ensuite d'une manière connue les groupes fonctionnels D en les groupes fonctionnels Z et Y de formule (I).

2. Composés de Z-stilbène nouveaux de formules

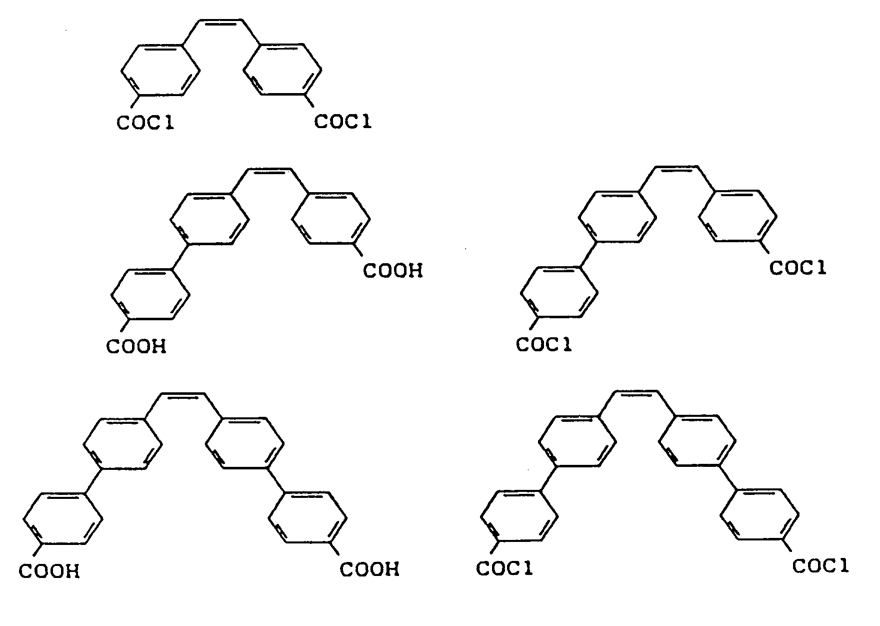

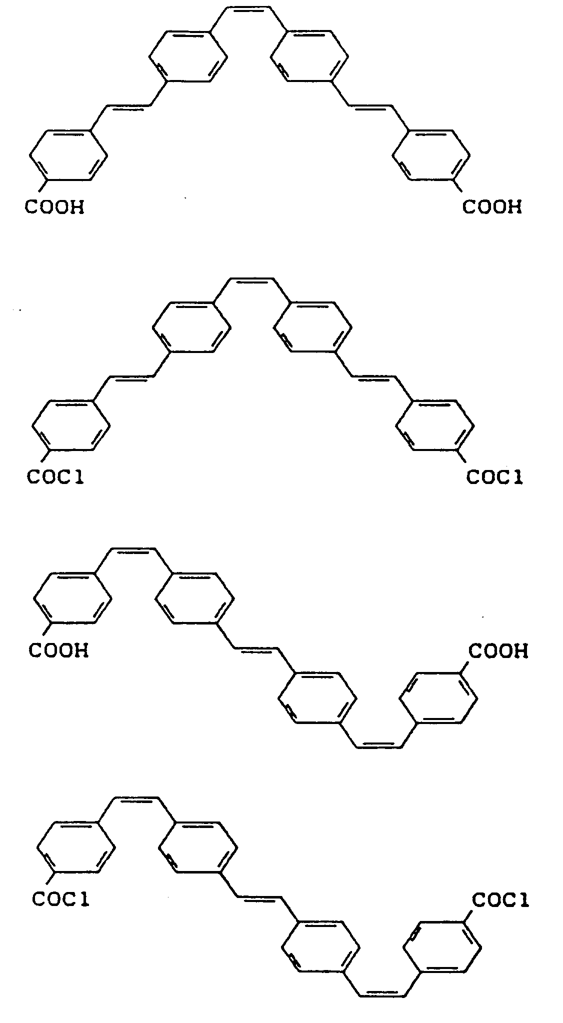

3. Utilisation des composés de Z-stilbène bifonctionnels obtenus conformément à la revendication 1 pour la production de polymères.
